# EUROPÄISCHE PATENTSCHRIFT

(11) **EP 1 965 753 B1**
(45) Veröffentlichungstag und Bekanntmachung des Hinweises auf die Patenterteilung: **04.09.2013**
(21) Anmeldenummer: 06829812.4
(22) Anmeldetag: 21.12.2006
(51) Int. Cl.: A61Q 5/06, A61K 8/33, A61K 8/49, A61Q 5/10

(54) **REVERSIBEL SCHALTBARE HAARFÄRBUNG**
REVERSIBLY CHANGEABLE HAIR COLOR
COLORATION CAPILLAIRE POUVANT VARIER DE FAÇON REVERSIBLE

(30) Priorität: 27.12.2005 DE 102005062834
(43) Veröffentlichungstag der Anmeldung: 10.09.2008
(73) Patentinhaber: Henkel AG & Co. KGaA, 40589 Düsseldorf (DE)
(72) Erfinder: OBERKOBUSCH, Doris, 40591 Düsseldorf (DE); HÖFFKES, Horst, 40595 Düsseldorf (DE); GROSS, Wibke, 40549 Düsseldorf (DE); MOCH, Melanie, 41542 Dormagen (DE)
(86) Internationale Anmeldenummer: PCT/EP2006/012379
(87) Internationale Veröffentlichungsnummer: WO 2007/073922

(56) Entgegenhaltungen:
- EP-A2- 1 300 134
- WO-A-2004/022016
- WO-A-2006/029687
- WO-A-2006/077039
- WO-A-2006/119819
- DE-U1- 29 908 573
- US-A1- 2005 210 605

## Beschreibung

Die Erfindung betrifft ein Mittel zum Färben von keratinhaltigen Fasern, insbesondere menschlichen Haaren, das spezielle CH-acide Verbindungen in Kombination mit ausgewählten Aldehyden als reaktive Carbonylverbindung enthält, die Verwendung dieser Kombination in Mitteln zum Erhalt einer schaltbaren Haarfärbung, sowie ein Verfahren zum Färben von keratinhaltigen Fasern, insbesondere menschlichen Haaren, und reversiblem Wechsel der Farbe ohne Verwendung farbgebender Substanzen.

Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsuntemehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Problematisch gestaltet sich nach wie vor eine Bereitstellung von Oxidationshaarfärbungen im Rotbereich mit ausreichenden Echtheitseigenschaften, insbesondere in mit sehr guten Wasch- und Reibechtheiten. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

In der Veröffentlichung H. Baumann et al., Liebigs Ann. Chem., 1968, 717, 124-136, werden Reaktionen von Pyrimidonen als Methylenbasen beschrieben. Ein Haarfärbemittel, enthaltend 1,2-Dihydro-2-oxo-pyrimidinium-Derivate, oder die Verwendung der offenbarten Hemicyanine zum Färben von keratinhaltigen Fasern wird hier nicht vorgeschlagen.

In der deutschen Patentanmeldung DE-A1-2047431 werden kationische Methinfarbstoffe zur Färbung von anionisch modifizierten Fasern, wie sauer modifizierten Polyestern oder Acrylnitrilpolymerisaten beschrieben. Zur Darstellung der kationischen Methinfarbstoffe werden unter anderem 3,4-Dihydro-3-methyl-4-methylenchinazol-2-on und 1,3,6-Trimethyl-4-methylen-pyrimidin-2-on sowie zwingend Terephthalaldehyd verwendet.

In der deutschen Patentanmeldung, DE-A1-2165913 wird ein Verfahren zur Herstellung von Ausbleichbildern unter Verwendung von lichtempfindlichen Farbstoffen vorgeschlagen. Die beanspruchten lichtempfindlichen Farbstoffe gehören zu der Klasse der Pyrimidon- bzw. Thiopyrimidonfarbstoffe.

In der deutschen Patentanmeldung DE-A1-102 41 076 werden 1,2-Dihydro-2-oxo-pyrimidinium-Derivate in Kombination mit reaktiven Carbonylverbindungen als Mittel zur Färbung von Keratinfasern vorgeschlagen.

Oftmals erscheint dem Verbraucher das Farbbild seiner Haare für einen bestimmten Anlass als unangemessen. Der Verbraucher schreckt in einer solchen Situation davor zurück, sich das Haar für jeden Anlass passend zu colorieren. Für eine farbenfrohe Maskerade, z.B. zu Karneval oder für einen Discobesuch, wünscht sich der Verbraucher eine Haarfärbung, die über einen gewissen Zeitraum unverändert bleibt und sich danach wieder in eine unauffällige Färbung oder bestenfalls in die Ausgangsfärbung zurückverwandeln lässt.

Überraschenderweise wurde nun gefunden, daß sich einerseits ausgewählte CH-acide Verbindungen in Kombination mit Benzaldehydderivaten hervorragend zum Färben von keratinhaltigen Fasern eignen. Sie ergeben Ausfärbungen mit hervorragender Brillanz und Farbtiefe und führen zu vielfältigen Farbnuancen. Es werden auch ohne den Einsatz von Oxidationsmitteln insbesondere Ausfärbungen mit verbesserten Wasch- und Lichtechtheitseigenschaften über einen Nuancenbereich von gelb über gelbbraun, orange, braunorange, braun, rot, rotviolett bis hin zu blauviolett, dunkelblau und schwarz erhalten. Durch eine Spülung, insbesondere ohne Einsatz vor farbgebenden Komponenten, lässt sich andererseits die erzielte Färbung in eine zweite Farbe ändern. Diese zweite Färbung kann durch eine erneute Spülung, insbesondere ohne Einsatz von farbgebenden Komponenten, wieder in die erste Farbe zurückverwandelt werden.

Ein erster Gegenstand der Erfindung ist eine Verpackungseinheit (Kit), enthaltend
- in einem Container 1a ein Mittel, enthaltend in einem kosmetischen Träger mindestens eine CH-acide Verbindung, ausgewählt aus der Gruppe, die gebildet wird aus Verbindungen gemäß Formel I und/oder deren Enaminformen, und aus Verbindungen der Formel (II), und Verbindungen der Formel (III) 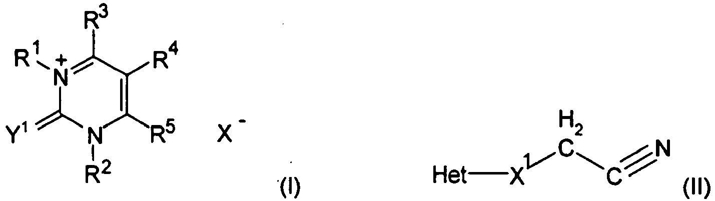 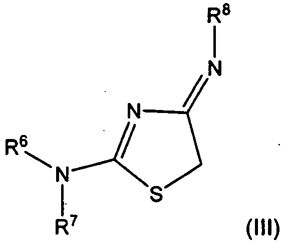 worin
   - R¹ und R² stehen unabhängig voneinander für eine lineare oder cyclische C₁-C₆-Alkylgruppe, eine C₂-C₆-Alkenylgruppe, eine gegebenenfalls substituierte Arylgruppe, eine gegebenenfalls substituierte Heteroarylgruppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxy-C₁-C₆-alkylgruppe, eine Gruppe R^{I}R^{II}N-(CH₂)ₚ-, worin R¹ und R^{II} stehen unabhängig voneinander für ein Wasserstoffatom, eine C₁-C₄-Alkylgruppe, eine C₁-C₄-Hydroxyalkylgruppe oder eine Aryl-C₁-C₆-alkylgruppe, wobei R^{I} und R^{II} gemeinsam mit dem Stickstoffatom einen 5-, 6- oder 7-gliedrigen Ring bilden können und p steht für eine Zahl 2, 3, 4, 5 oder 6,
   - R³ und R⁵ stehen unabhängig voneinander für ein Wasserstoffatom oder eine C₁-C₆-Alkylgruppe, wobei mindestens einer der Reste R³ oder R⁵ eine C₁-C₆-Alkylgruppe bedeutet,
   - R⁴ steht für ein Wasserstoffatom, eine C₁-C₆-Alkylgruppe, eine C₁-C₆-Hydroxyalkylgruppe, eine C₂-C₆-Polyhydroxyalkylgruppe, eine C₁-C₆-Alkoxygruppe, eine C₁-C₆-Hydroxyalkoxygruppe, eine Gruppe R^{III}RI^{V}N-(CH₂)_{q}-, worin R^{III} und R^{IV} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und q steht für eine Zahl 1, 2, 3, 4, 5 oder 6, wobei der Rest R⁴ zusammen mit einem der Reste R³ oder R⁵ einen 5- oder 6-gliedrigen aromatischen Ring bilden kann, der gegebenenfalls mit einem Halogenatom, einer C₁-C₆-Alkylgruppe, einer C₁-C₆-Hydroxyalkylgruppe, einer C₂-C₆-Polyhydroxyalkylgruppe, einer C₁-C₆-Alkoxygruppe, einer C₁-C₆-Hydroxyalkoxygruppe, einer Nitrogruppe, einer Hydroxygruppe, einer Gruppe R^{V}R^{VI}N-(CH₂)ₛ-, worin R^{V} und R^{VI} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und s steht für eine Zahl 0, 1, 2, 3, 4, 5 oder 6 substituiert sein kann,
   - Y¹ steht für ein Sauerstoffatom, ein Schwefelatom oder eine Gruppe NR^{VII}, worin R^{VII} steht für ein Wasserstoffatom, eine Arylgruppe, eine Heteroarylgruppe, eine C₁-C₆-Alkylgruppe oder eine C₁-C₆-Arylalkylgruppe,
   - X⁻ steht für ein physiologisch verträgliches Anion,
   - Het steht für einen gegebenenfalls substituierten Heteroaromaten,
   - X¹ steht für eine direkte Bindung oder eine Carbonylgruppe.
   - R⁶ und R⁷ bilden entweder zusammen mit dem Stickstoffatom einen gesättigten oder ungesättigten 5- oder 6-gliedrigen Ring oder stehen unabhängig voneinander für eine (C₁-C₆)-Alkylgruppe, eine (C₂-C₆)-Alkenylgruppe, eine Arylgruppe, eine Aryl-(C₁-C₆)-alkylgruppe, eine (C₂-C₆)-Hydroxyalkylgruppe, eine (C₂-C₆)-Polyhydroxyalkylgruppe oder eine Gruppe R^{I}R^{II}N-(CH₂)ₘ-, worin R^{I} und R^{II} stehen unabhängig voneinander für ein Wasserstoffatom, eine (C₁-C₆)-Alkylgruppe, eine (C₁-C₆)-Alkenylgruppe oder eine Aryl-C₁-C₆-alkylgruppe, wobei R^{I} und R^{II} gemeinsam mit dem Stickstoffatom einen 5-, 6- oder 7-gliedrigen Ring bilden können und m steht für eine Zahl 2, 3, 4, 5 oder 6, und
   - R⁸ ein Wasserstoffatom, eine (C₁-C₆)-Alkylgruppe, eine (C₂-C₆)-Alkenylgruppe, eine Arylgruppe, eine Aryl-(C₁-C₆)-alkylgruppe, eine (C₂-C₆)-Hydroxyalkylgruppe, eine (C₂-C₆)-Polyhydroxyalkylgruppe oder eine Gruppe R^{III}R^{IV}N-(CH₂)ₙ-, worin R^{III} und R^{IV} stehen unabhängig voneinander für ein Wasserstoffatom, eine (C₁-C₆)-Alkylgruppe, eine (C₁-C₆)-Alkenylgruppe oder eine Aryl-C₁-C₆-alkylgruppe, wobei R^{III} und R^{IV} gemeinsam mit dem Stickstoffatom einen 5-, 6- oder 7-gliedrigen Ring bilden können und n steht für eine Zahl 2, 3, 4, 5 oder 6,
- in einem Container 1b ein Mittel, enthaltend in einem kosmetischen Träger mindestens einen Aldehyd gemäß Formel (IV) 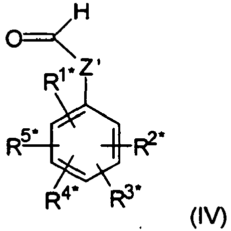 worin
   - R^{1*}, R^{2*} und R^{3*} stehen unabhängig voneinander für ein Wasserstoffatom, ein Halogenatom, eine C₁-C₆-Alkylgruppe, eine Hydroxygruppe, eine C₁-C₆-Alkoxygruppe, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxygruppe, eine Sulfonsäuregruppe, eine Sulfonamidogruppe, eine Sulfonamidgruppe, Carbamoylgruppe, eine C₂-C₆-Acylgruppe oder eine Nitrogruppe,
   - Z' steht für eine direkte Bindung oder eine Vinylengruppe,
   - R^{4*} und R^{5*} stehen für ein Wasserstoffatom oder bilden gemeinsam, zusammen mit dem Restmolekül einen 5- oder 6-gliederigen aromatischen oder aliphatischen Ring.
- in einem Container 2 ein kosmetisches Mittel mit einem sauren pH-Wert und
- in einem Container 3 ein kosmetisches Mittel mit einem alkalischen pH-Wert.

Geeignete kosmetische Träger für alle Mittel des Kits sind generell z.B. Cremes, Emulslonen, Gele oder auch tensidhaltige schäumende Lösungen, wie beispielswelse Shampoos, Schaumaerosole oder andere Zubereitungen, die insbesondere für die Anwendung auf dem Haar geeignet sind, Es ist aber auch denkbar, die Inhaltsstoffe In eine pulverförmige oder auch tablettenförmlge Formullerung zu integrieren, welche vor der Anwendung in Wasser gelöst wird. Bevorzugt sind Cremes, Emulsionen und Gele.

Die Träger sind insbesondere wässrig oder wässrig-alkoholisch.

Ein wässriger Träger enthält mindestens 50 Gew.-% Wasser.

Unter wässrig-alkoholischen Trägern sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel,

Vorzugsweise werden die Verbindungen gemäß Formel I ausgewählt aus einer oder mehrerer Verbindungen der Gruppe von Salzen mit physiologisch verträglichem Gegenion X⁻, die gebildet wird aus Salzen des
1,2-Dihydro-1,3,4,6-tetramethyl-2-oxo-pyrimidiniums,
1,2-Dihydro-1,3-diethyl-4,6-dimethyl-2-oxo-pyrimidiniums,
1,2-Dihydro-1,3-dipropyl-4,6-dimethyl-2-oxo-pyrimidiniums,
1,2-Dihydro-1,3-di(2-hydroxyethyl)-4,6-dimethyl-2-oxo-pyrimidiniums,
1,2-Dihydro-1,3-diphenyl-4,6-dimethyl-2-oxo-pyrimidiniums,
1,2-Dihydro-1,3,4-trimethyl-2-oxo-pyrimidiniums,
1,2-Dihydro-1,3-diethyl-4-methyl-2-oxo-pyrimidiniums,
1,2-Dihydro-1,3-dipropyl-4-methyl-2-oxo-pyrimidiniums,
1,2-Dihydro-1,3-di(2-hydroxyethyl)-4-methyl-2-oxo-pyrimidiniums,
1,2-Dihydro-1,3-diphenyl-4-methyl-2-oxo-pyrimidiniums,
1-Allyl-1,2-dihydro-3,4,6-trimethyl-2-oxo-pyrimidiniums,
1,2-Dihydro-1-(2-hydroxyethyl)-3,4,6-trimethyl-2-oxo-pyrimidiniums,
1,2-Dihydro-1,3,4,6-tetramethyl-2-thioxo-pyrimidiniums,
1,2-Dihydro-1,3-diethyl-4,6-dimethyl-2-thioxo-pyrimidiniums,
1,2-Dihydro-1,3-dipropyl-4,6-dimethyl-2-thioxo-pyrimidiniums,
1,2-Dihydro-1,3-di(2-hydroxyethyl)-4,6-dimethyl-2-thioxo-pyrimidiniums,
1,2-Dihydro-1,3-diphenyl-4,6-dimethyl-2-thioxo-pyrimidiniums,
1,2-Dihydro-1,3,4-trimethyl-2-thioxo-pyrimidiniums,
1,2-Dihydro-1,3-diethyl-4-methyl-2-thioxo-pyrimidiniums,
1,2-Dihydro-1,3-dipropyl-4-methyl-2-thioxo-pyrimidiniums,
1,2-Dihydro-1,3-di(2-hydroxyethyl)-4-methyl-2-thioxo-pyrimidiniums,
1,2-Dihydro-1,3-diphenyl-4-methyl-2-thioxo-pyrimidiniums,
1,2-Dihydro-3,4-dimethyl-2-oxo-chinazoliniums und
1,2-Dihydro-3,4-dimethyl-2-thioxo-chinazoliniums.

Die physiologisch verträglichen Anionen X- gemäß Formel (I) bzw. oben genannter Liste dürfen definitionsgemäß nicht nur eine negative Ladung tragen, sondern können auch eine Ladungszahl größer 1 besitzen. In letzterem Falle werden die Anionen X- der Salzform zur Wahrung der Elektroneutralität durch Formulierung eines stöchiometrischen Koeffizienten kleiner 1 vor der Bezeichnung des Anions beschrieben. Die physiologisch verträglichen Anionen werden bevorzugt ausgewählt aus Halogenid, 0.5 Sulfat, Hydrogensulfat, 0.5 Carbonat, Hydrogencarbonat, 1/3 Phosphat, 0.5 Hydrogenphosphat, Dihydrogenphosphat, Carboxylat, wie beispielsweise Lactat, Citrat oder Tartrat. Besonders bevorzugt steht X- für Chlorid, Bromid oder ein Carboxylat-Gegenion, insbesondere für Lactat, Citrat oder Tartrat.

Wenn es sich bei den erfindungsgemäßen Verbindungen mit der Formel (II) um stickstoffatomhaltige Verbindungen handelt, können aus diesen in vielen Fällen in üblicher Weise die bekannten Salze als Säureadditionssalze hergestellt werden. Alle Aussagen dieser Schrift und demgemäss der beanspruchte Schutzbereich beziehen sich daher sowohl auf die in freier Form vorliegenden Verbindungen als auch auf deren wasserlösliche, physiologisch verträgliche Salze. Beispiele für solche Salze sind die Hydrochloride, die Hydrobromide, die Sulfate, die Phosphate, die Acetate, die Propionate, die Citrate und die Lactate. Die Hydrochloride und die Sulfate sind dabei besonders bevorzugt. Gleiches gilt für die Aminogruppen-haltigen Verbindungen gemäß Formel (III).

Erfindungsgemäß eignen sich Verbindungen gemäß Formel (II) besonders gut solche, in denen sich der Rest Het gemäß Formel II ableitet von einem der Heteroaromaten Furan, Thiophen, Pyrrol, Isoxazol, Isothiazol, Imidazol, Oxazol, Thiazol, Pyridin, Pyridazin, Pyrimidin, Pyrazin, 1,2,3-Triazin, 1,2,4-Triazin, 1,3,5-Triazin, Benzopyrrol, Benzofuran, Benzothiophen, Benzimidazol, Benzoxazol, Indazol, Benzoisoxazol, Benzoisothiazol, Indol, Chinolin, Isochinolin, Cinnolin, Phthalazin, Chinazolin, Chinoxalin, Acridin, Benzochinolin, Benzoisochinolin, Phenazin, Benzocinnolin, Benzochinazolin, Benzochinoxalin, Phenoxazin, Phenothiazin, Nephthyridin, Phenanthrolin, Indolizin, Chinolizin, Carbolin, Purin, Pteridin und Cumarin, wobei die vorgenannten Heteroaromaten mit mindestens einer Gruppe ausgewählt aus einem Halogenatom, einer Nitrogruppe, einer Thiogruppe, einer Thio-(C₁-C₆)-alkylgruppe, einer Heteroarylgruppe, einer Arylgruppe, einer (C₁-C₆)-Alkylgruppe, einer (C₁-C₆)-Alkoxygruppe, einer Hadroxygruppe, einer (C₂-C₆)-Hydroxyalkylgruppe, einer (C₂-C₆)-Polyhydroxyalkylgruppe, einer (C₁-C₆)-Alkoxyl-(C₁-C₆)-alkylgruppe, einer Aryl-(C₁-C₆)-alkylgruppe, einer Aminogruppe, einer (C₁-C₆)-Monoalkylaminogruppe, einer (C₁-C₆)-Dialkylaminogruppe, eine Dialkylaminoalkylgruppe -(CH₂)ₙ-NR'R", worin n eine ganze Zahl von 2 und 6 ist und R' und R" unabhängig voneinander eine lineare oder verzweigte Alkylgruppe bedeutet, welche gegebenenfalls zusammen einen Ring bilden können, substituiert sein können.

Vorzugsweise werden die Verbindungen gemäß Formel II ausgewählt aus mindestens einem Vertreter der Gruppe bestehend aus 2-(2-Furoyl)-acetonitril, 2-(5-Brom-2-furoyl)-acetonitril, 2-(5-Methyl-2-trifluormethyl-3-furoyl)-acetonitril, 3-(2,5-Dimethyl-3-furyl)-3-oxopropanitril, 2-(2-Thenoyl)-acetonitril, 2-(3-Thenoyl)-acetonitril, 2-(5-Fluor-2-thenoyl)-acetonitril, 2-(5-Chlor-2-thenoyl)-acetonitril, 2-(5-Brom-2-thenoyl)-acetonitril, 2-(5-Methyl-2-thenoyl)-acetonitril, 2-(2,5-Dimethylpyrrol-3-oyl)-acetonitril, 2-(1,2,5-Trimethylpyrrol-3-oyl)-acetonitril, 1*H*-Benzimidazol-2-ylacetonitril, 1*H*-Benzothiazol-2-ylacetonitril, 2-(Pyrid-2-yl)-acetonitril, 2,6-Bis(cyanmethyl)-pyridin, 2-(Indol-3-oyl)-acetonitril, 2-(2-Methyl-indol-3-oyl)-acetonitril, 8-Cyanacetyl-7-methoxy-4-methylcumarin, 2-(2-Isopropyl-5,6-benzochinolin-4-oyl)-acetonitril, 2-(2-Phenyl-5,6-benzochinolin-4-oyl)-acetonitril, 2-(Chinoxalin-2-yl)-acetonitril, 2-(Cumaron-2-yl)-acetonitril, 6,7-Dichlor-5-(cyanoacetyl)-2,3-dihydro-1-benzofuran-2-carbonsäure-tert.-butylester, 2-(6-Hydroxy-4,7-dimethoxy-1-benzofuran-5-oyl)-acetonitril und 2-(1-Phenyl-1,4-dihydrothiochromeno[4,3-c]pyrazol-3-oyl)-acetonitril.

Bevorzugt geeignete Verbindungen der Formel (III) werden aus den Vertretern ausgewählt, bei denen die Reste R⁶ und R⁷ gemäß Formel (III) zusammen mit dem Stickstoffatom einen gesättigten 5- oder 6-gliedrigen Ring bilden. Dieser Ring wiederum kann gegebenenfalls ein Sauerstoffatom und/oder gegebenenfalls mehrere Stickstoffatome im Gerüst enthalten. Besonders bevorzugte Beispiele für solche Ringe sind Piperidinyl, Morpholinyl und Pyrrolidinyl.

Erfindungsgemäß ganz besonders bevorzugte Verbindungen gemäß Formeln (I), (II) und Formel (III) werden ausgewählt aus mindestens einer der nachfolgenden Verbindungen

| | **Struktur** |
|---|---|
| Salze des | 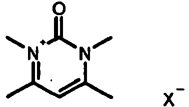 |
| 1,2-Dihydro-1,3,4,6-tetramethyl-2-oxopyrimidiniums | |
| Salze des | 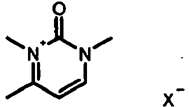 |
| 1,2-Dihydro-1,3,4-trimethyl-2-oxopyrimidiniums | |
| Salze des | 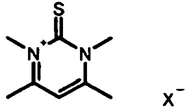 |
| 1,2-Dihydro-1,3,4,6-tetramethyl-2-thioxopyrimidiniums | |
| Salze des | 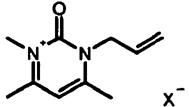 |
| 1-Allyl-1,2-dihydro-3,4,6-trimethyl-2-oxopyrimidiniums | |
| Salze des | 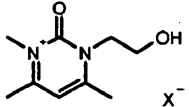 |
| 1,2-Dihydro-1-(2-hydroxyethyl)-3,4,6-trimethyl-2-oxopyrimidiniums | |
| | 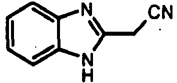 |
| 2-(Cyanomethyl)benzimidazol | |
| | 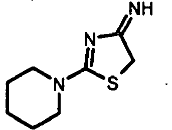 |
| 4,5-Dihydro-4-imino-2-(1-piperidinyl)-thiazol | |
| | 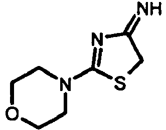 |
| 4,5-Dihydro-4-imino-2-(4-morpholinyl)-thiazol | |
| | 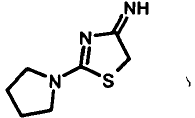 |
| 4,5-Dihydro-4-imino-2-(1-pyrrolidinyl)-thiazol | |

wobei die Salze der vorgenannten Verbindungen X⁻ als ein physiologisch verträgliches Gegenion enthalten. Ganz besonders bevorzugte Verbindungen der Formel (I) sind Salze des 1,2-Dihydro-1,3,4,6-tetramethyl-2-oxopyrimidiniums (insbesondere mit X⁻ = hydrogensulfat), des 1-Allyl-1,2-dihydro-3,4,6-trimethyl-2-oxopyrimidiniums (insbesondere mit X⁻ = bromid) sowie des 1,2-Dihydro-1-(2-hydroxyethyl)-3,4,6-trimethyl-2-oxopyrimidiniums (insbesondere mit X- = p-Toluolsulfonat).

Als CH-acide Verbindungen werden im Allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Die erfindungsgemäßen Verbindungen gemäß Formeln I II und III sind CH-acide Verbindungen.
Die Verbindungen der Formel (I) liegen im chemischen Gleichgewicht mit ihrer korrespondierenden Enaminform vor. Mit Hilfe einer Base lassen sich aus den Verbindungen der besagten Formeln durch Deprotonierung am Kohlenstoffatom der aktivierten Methylgruppen in 4- oder 6-Position die entsprechenden Enamine gezielt darstellen. Exemplarisch wird diese Deprotonierung nachfolgend am Rest R³ der Formel I illustriert. Verbindungen gemäß Formel Ia sind Beispiele für die erfindungsgemäßen Enaminformen der erfindungsgemäßen Verbindungen gemäß Formel (I). Eine vergleichbare Deprotonierung am Rest R⁵ der Formel (I) ist ebenso möglich

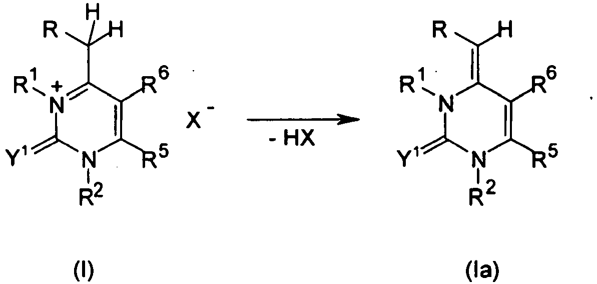

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose verwendet werden.

Die Verbindungen der Formel (IV) werden bevorzugt ausgewählt aus mindestens einer Verbindung der Gruppe 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethytamino-2-hydroxybenzaldehyd, 4-Dimethylamino-2-methoxybenzaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxybenzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxybenzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxybenzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 3,5-Dichlor-4-hydroxybenzaldehyd, 4-Hydroxy-3,5-diiod-benzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 4-Hydroxy-3-iod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 3-Hydroxy-4-nitrobenzaldehyd, 2-Hydroxy-3-methoxy-5-nitrobenzaldehyd, 5-Nitrovanillin, 2,5-Dinitrosalicylaldehyd, 5-Brom-3-nitrosalicylaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylaminobenzaldehyd, 3-Allyl-4-hydroxybenzaldehyd, 3-Allyl-4-hydroxy-5-methoxybenzaldehyd, 3-Allyl-4-hydroxy-5-methylbenzaldehyd, 3-Allyl-5-brom-4-hydroxybenzaldehyd, 3,5-Diallyl-4-hydroxybenzaldehyd, 3-Allyl-4-hydroxy-5-formylbenzaldehyd (5-Allyl-4-hydroxyisophthalaldehyd) und Piperonal.

Als im Sinne der Erfindung besonders bevorzugt geeignete Verbindungen der Formel (III) eignet sich mindestens ein Vertreter der Gruppe, die gebildet wird, aus 4-Hydroxy-3-methoxybenzaldehyd (Vanillin), 3-Ethoxy-4-hydroxybenzaldeyd (Ethylvanillin), 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 3-Brom-4-hydroxybenzaldehyd, 4-Hydroxy-3-methyl-benzaldehyd, 3,5-Dimethyl-4-hydroxybenzaldehyd und 5-Brom-4-hydroxy-3-methoxybenzadlehyd (5-Bromvanillin).

Ganz besonders bevorzugt eignet sich mindestens einer der folgenden Verbindungen 4-Hydroxy-3-methoxybenzaldehyd (Vanillin), 3-Ethoxy-4-hydroxybenzaldeyd (Ethylvanillin), 3,5-Dimethoxy-4-hydroxybenzaldehyd, 4-Hydroxy-1-naphthaldehyd und 4-Hydroxy-2-methoxybenzaldehyd als Verbindung gemäß Formel (IV)
Diese bevorzugten bzw. besonders bevorzugten Vertreter der Verbindungen gemäß Formel (IV) werden wiederum bevorzugt mit den zuvor erwähnten bevorzugten Verbindungen der Formeln (I) und/oder (II) und/oder (III) kombiniert.

Es ist erfindungsgemäß bevorzugt, dass die Mittel des Containers 1a und 1 b, bzw. deren Mischung keine weiteren farbgebenden Komponenten enthalten.

Bevorzugt enthält das Mittel mit einem sauren pH-Wert keine farbgebenden Komponenten.

Das kosmetische Mittel mit einem sauren pH-Wert besitzt bevorzugt einen pH-Wert von pH 2 bis pH 6. Im Allgemeinen ist es bevorzugt, dass dieses Mittel zusätzlich mindestens ein Puffersystem enthält. Dieses dient der Stabilisierung des sauren pH-Wertes während der Lagerung und Anwendung. Der Auswahl des Puffersystems für einen sauren pH-Wert sind keine Grenzen gesetzt.

Bevorzugt enthält das Mittel mit einem alkalischen pH-Wert keine farbgebenden Komponenten.

Das kosmetische Mittel mit einem alkalischen pH-Wert besitzt bevorzugt einen pH-Wert von pH 8 bis pH 11. Im Allgemeinen ist es bevorzugt, dass dieses Mittel zusätzlich mindestens ein Puffersystem enthält. Dieses dient der Stabilisierung des pH-Wertes während der Lagerung und Anwendung. Der Auswahl des Puffersystems für einen alkalischen pH-Wert sind keine Grenzen gesetzt.

Farbgebende Komponenten sind im Sinne der Erfindung Bestandteile eines Mittels, die, wenn das betreffende Mittel auf keratinhaltige Fasern aufgetragen wird, eine mit dem Auge sichtbare Farbveränderung dieser keratinhaltigen Fasern bewirken. Farbstoffe, die das Mittel anfärben, aber keine Anfärbung der keratinhaltigen Fasern bewirken können in den Mitteln des Containers 2 und des Containers 3 enthalten sein.

Die kosmetischen Mittel der Container 2 und 3 enthalten einen kosmetischen Träger und mindestens ein pH-Stellmittel.

Geeignete pH-Stellmittel sind für die Einstellung eines sauren pH-Wertes bevorzugt Carbonsäuren, insbesondere Genußsäuren (wie z.B. Weinsäure, Zitronensäure, Äpfelsäure oder Milchsäure), Phosphorsäure, Schwefelsäure oder HalogenwasserstoffSäuren (wie z.B. Salzsäure).

Geeignete pH-Stellmittel sind für die Einstellung eines alkalischen pH-Wertes bevorzugt Ammoniak, Alkalihydroxide (wie z.B. Natriumhydroxyid oder Kaliumhydroxyid), Alkanolamine oder basische Aminosäure, wie beispielsweise Arginin oder Lysin.

Unter dem Begriff Alkanolamin sind erfindungsgemäß organische Aminverbindungen zu verstehen, die mindestens eine C₂- bis C₆-Hydroxyalkylgruppe tragen. Die C₂- bis C₆-Hydroxyalkylgruppe trägt wiederum mindestens eine Hydroxygruppe. Bei den erfindungsgemäßen Alkanolaminen handelt es sich bevorzugt um primäre Amine.

Als C₂- bis C₆-Hydroxyalkylgruppe fungieren beispielsweise 2-Hydroxyethyl, 1,3-Dihydroxy-2-methyl-propan-2-yl, 2-Ethyl-1,3-dihxdroxy-propan-2-yl, 1-Hydroxy-2-methylbutan-2-yl, 3-Hydroxypropyl und 4-Hydroxybutyl.

Alkanolamine werden bevorzugt aus mindestens einem Vertreter der Gruppe ausgewählt, die von 2-Aminoethanol (Monoethanolamin), Monoisopropanolamin, 2-Amino-2-methyl-propanol, 2-Amino-2-methyl-1,3-propandiol, 2-Amino-2-ethyl-1,3-propandiol und 2-Amino-2-methylbutanol gebildet wird, besonders bevorzugt ausgewählt aus mindestens einem Vertreter der Gruppe, die Monoethanolamin, 2-Amino-2-methyl-propanol und 2-Amino-2-methyl-1,3-propandiol umfaßt.

Zur Erlangung weiterer und intensiverer Ausfärbungen können die erfindungsgemäßen Mittel des Containers 1a und/oder 1b zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Arginin, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65 mmol, insbesondere 1 bis 40 mmol, jeweils bezogen auf 100 g des gesamten Mittels, eingesetzt werden.

Die erfindungsgemäßen Färbemittel werden vor der Anwendung aus dem Mittel des Containers 1a und 1b gemischt. Sie ergeben bereits bei physiologisch verträglichen Temperaturen von unter 45°C intensive Färbungen. Sie eignen sich deshalb besonders zum Färben von menschlichen Haaren.

Auf die Anwesenheit von Oxidationsmitteln, z. B. H₂O₂, in mindestens einem der Mittel des Containers 1a oder 1 b kann verzichtet werden. Es kann jedoch u. U. wünschenswert sein, dem anwendungsbereiten Färbemittel zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂. Auch Gemische von mehreren Oxidationsmitteln, wie beispielsweise eine Kombination aus Wasserstoffperoxid und Peroxodisulfaten der Alkali- und Erdalkalimetalle oder aus Iodidionenquellen, wie z.B. Alkalimetalliodiden und Wasserstoffperoxid oder den vorgenannten Peroxodisulfaten, können verwendet werden. Das Oxidationsmittel bzw. die Oxidationsmittelkombihation können erfindungsgemäß in Verbindung mit Oxidationskatalysatoren in dem Haarfärbemittel zur Anwendung kommen. Oxidationskatalysatoren sind beispielsweise Metallsalze, Metallchelat-Komplexe oder Metalloxide, die einen leichten Wechsel zwischen zwei Oxidationsstufen der Metallionen ermöglichen. Beispiele sind Salze, Chelatkomplexe oder Oxide von Eisen, Ruthenium, Mangan und Kupfer. Weitere mögliche Oxidationskatalysatoren stellen Enzyme dar. Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Weiterhin können die erfindungsgemäßen Mittel aus den Containern 1a, 1 b, 2 und 3 alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe, .
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester
- Anlagerungsprodukte von Ethylenoxid an Fettsäurealkanolamide.

Beispiele für die in den erfindungsgemäßen Mitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquatemäre Polydimethylsiloxane, Quatemium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat^{®}100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des kosmetischen Trägers werden zur Herstellung der Mittel der erfindungsgemäßen Verpackungseinheit in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, mindestens einem Mittel der Container C1a oder C1b Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65 mmol, insbesondere von 1 bis 40 mmol, bezogen auf 100 g der Anwendungsmischung, enthalten.

Der pH-Wert der Anwendungsmischung aus den Mitteln des Containers C1 a und C1b liegt üblicherweise zwischen 2 und 11, vorzugsweise zwischen 8 und 10.

Ein zweiter Gegenstand der Erfindung ist ein Verfahren zur reversiblen Umfärbung keratinhaltiger Fasern, insbesondere menschlicher Haare, welche zuvor mit einem Färbemittel, enthaltend in einem kosmetischen Träger eine Kombination aus Komponente

### (A) mindestens einer Verbindung der Formel (I) und/oder (II) und/oder (III)

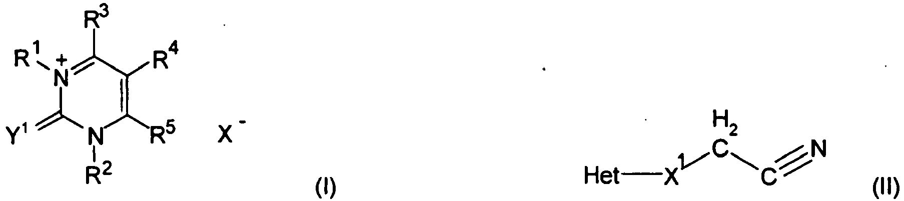

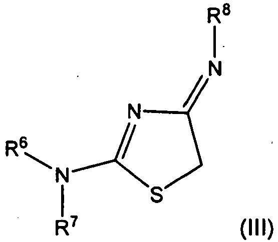

worin R¹, R², R³, R⁴, R⁵, R⁶, R⁷, R⁸, Het, X¹ und Y¹ wie im ersten Erfindungsgegenstand definiert sind
mit Komponente (B) mindestens einer Verbindung gemäß Formel (IV) 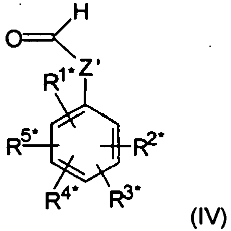 worin R^{1*}, R^{2*}, R^{3*}, R^{4*}, R^{5*} und Z' wie im ersten Erfindungsgegenstand beschrieben definiert sind,
gefärbt wurden, und
(a) die gefärbten keratinhaltigen Fasern mit einem kosmetischen Mittel mit einem sauren pH-Wert umgefärbt werden und
(b) gegebenenfalls nach einem Zeitraum von bis zu 4 Wochen mit einem kosmetischen Mittel mit einem alkalischen pH-Wert wieder umgefärbt werden.

Es ist bevorzugt, wenn das Färbemittel des erfindungsgemäßen Verfahrens einen pH-Wert von pH 6 bis pH 11, besonders bevorzugt einen pH-Wert von 8 bis 10, besitzt.

Zum Einsatz in dem Färbemittel eignen sich besonders die bevorzugten Vertreter der Verbindungen der Formeln (I), (II), (III) und (IV) gemäß erstem Erfindungsgegenstand. Alle bevorzugten Ausführungsformen der Mittel der Container 1a und 1b, bzw. deren Mischung die im ersten Erfindungsgegenstand erwähnt werden, gelten für das Färbemittel des erfindungsgemäßen Verfahrens.

Das kosmetische Mittel mit einem sauren pH-Wert gemäß Schritt (a) des erfindungsgemäßen Verfahrens hat einen bevorzugten pH-Wert von pH 2 bis pH 6.

Es gelten für das kosmetische Mittel aus Schritt (a) die bevorzugten und optionalen Parameter des Mittels in Container 2 des ersten Erfindungsgegenstandes.

Das kosmetische Mittel mit einem alkalischen pH-Wert gemäß Schritt (b) des erfindungsgemäßen Verfahrens hat einen bevorzugten pH-Wert von pH 8 bis pH 11.

Es gelten für das kosmetische Mittel aus Schritt (b) die bevorzugten und optionalen Parameter des Mittels in Container 3 des ersten Erfindungsgegenstandes.

Nach Durchführung des Schritts (b) wird bevorzugt die Färbung bzw. der Farbton erzielt, der vor Durchführung des Schritts (a) vorlag.

Die Schritte (a) und (b) können mehrmals durchlaufen werden.

Die zuvor mit dem besagten Färbemittel gefärbten Fasern können zu einem beliebigen Zeitpunkt vor Durchführung des Schritts (a) gefärbt worden sein. Wichtig ist, dass eine sichtbare Färbung durch das besagte Färbemittel existiert, so dass eine effektive Farbänderung gemäß Schritt (a) erfolgen kann.

Gemäß Schritt (a) bzw. Schritt (b) wird das entsprechende kosmetische Mittel auf die nasse oder trockene keratinhaltige Faser aufgetragen Die Mittel der Schritte (a) bzw. (b) können nach dem Auftragen auf die gefärbte keratinhaltige Faser entweder auf der Faser belassen werden (leave on) oder von der Faser heruntergespült werden. Es ist erfindungsgemäß bevorzugt, die jeweiligen Schritte (a) bzw. (b) des erfindungsgemäßen Verfahrens ohne abschließenden Spülvorgang durchzuführen, um die entsprechenden Mittel auf dem Haar zu belassen.

Ein dritter Gegenstand der Erfindung ist die Verwendung eines kosmetischen Mittels mit einem sauren pH-Wert zur Farbänderung keratinhaltiger Fasern, insbesondere menschlicher Haare, die mit einem Färbemittel, enthaltend in einem kosmetischen Träger eine Kombination aus Komponente

### (A) mindestens einer Verbindung der Formel (I) und/oder (II) und/oder (III)

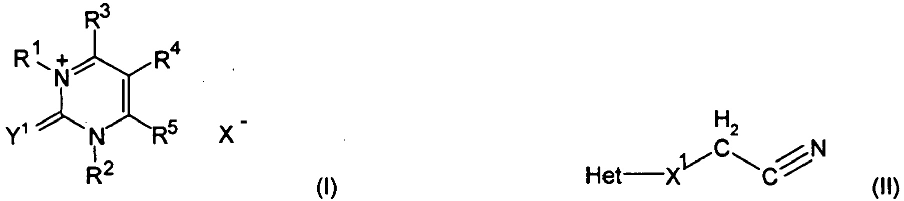

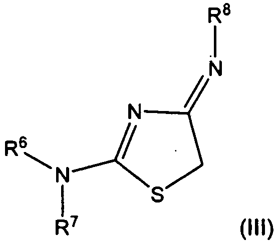

worin R¹, R², R³, R⁴, R⁵, R⁶, R⁷, R⁸, Het, X¹ und Y¹ wie im ersten Erfindungsgegenstand definiert sind
mit Komponente (B) mindestens einer Verbindung gemäß Formel (IV) 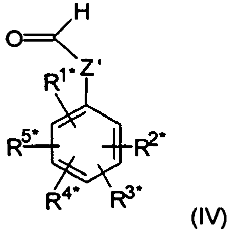 worin R^{1*}, R^{2*}, R^{3*}, R^{4*}, R^{5*} und Z' wie im ersten Erfindungsgegenstand beschrieben definiert sind,
gefärbt wurden.

Ein vierter Gegenstand ist die Verwendung eines kosmetischen Mittels mit einem alkalischen pH-Wert zur Wiederherstellung einer Färbung keratinhaltiger Fasern, insbesondere menschlicher Haare, die zunächst mit einem Färbemittel, enthaltend in einem kosmetischen Träger eine Kombination aus Komponente

### (A) mindestens einer Verbindung der Formel (I) und/oder (II) und/oder (III)

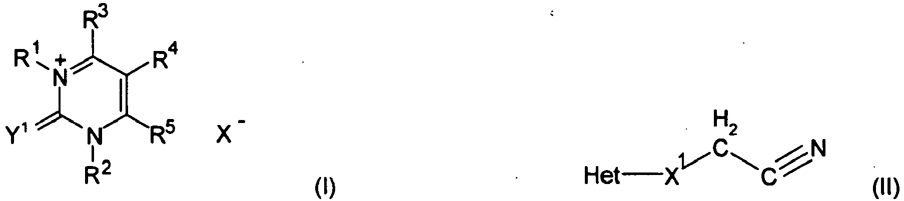

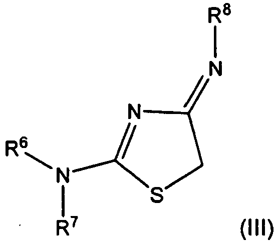

worin R¹, R², R³, R⁴, R⁵, R⁶, R⁷, R⁸, Het, X¹ und Y¹ wie im ersten Erfindungsgegenstand definiert sind
mit Komponente (B) mindestens einer Verbindung gemäß Formel (IV) 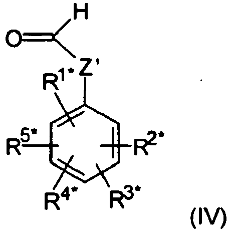 worin R^{1*}, R^{2*}, R^{3*}, R^{4*}, R^{5*} und Z' wie im ersten Erfindungsgegenstand beschrieben definiert sind,
gefärbt wurden und dann mit einem kosmetischen Mittel mit saurem pH-Wert einer Farbveränderung unterworfen wurden.

Alle bevorzugten und optionalen Parameter des ersten und zweiten Erfindungsgegenstandes gelten mutatis mutandis auch für die Erfindungsgegenstände drei und vier.

### Beispiele

### 1.0 Herstellung der Färbemittel

| Wässrige Gelformulierung für Komponente A | Gel 1: |
|---|---|
| | |
| CH-acide Verbindung (Komponente A) | 10 mmol |
| Natrosol^{®} HR 250 | 2 g |
| Wasser, vollentsalzt | ad 100 g |

Die CH-acide Verbindung (Komponente A) wird zunächst unter Rühren in wenig Wasser gelöst, dann wird mit Wasser auf 98 g aufgefüllt. Unter Rühren wird das Natrosol (INCI-Bezeichnung: Hydroxyethylcellulose; Firma Hercules) zugegeben und gewartet, bis es zur gewünschten Verdickung kommt.

| Wässrige Gelformulierung für Komponente B | Gel 2: |
|---|---|
| | |
| Carbonylverbindung (Komponente B) | 10 mmol |
| Natrosol^{®} HR 250 | 2 g |
| NaOH (50%ige wässrige Lösung) | evtl. einige Tropfen |
| Wasser, vollentsalzt | ad 100 g |

Die Carbonylverbindung (Komponente B) wird in wenig Wasser gelöst bzw. suspendiert. Zur Erhöhung der Löslichkeit wird bei Bedarf mit einigen Tropfen 50%iger Natronlauge alkalisiert. Anschließend wird mit Wasser auf 98 g aufgefüllt und bis zur vollständigen Lösung der Carbonylverbindung gerührt (teilweise unter gelindem Erwärmen auf ca. 40 °C). Anschließend wird unter Rühren das Natrosol hinzugegeben und der Quellvorgang abgewartet.

### 2.0 Ausfärbungen

Es wurden wässrige Gelformulierungen aus Punkt 1.0 (Gel 1 und Gel 2) mit den Komponenten A und B gemäß Tabelle 1 hergestellt. Die Gele wurden im Gewichtsverhältnis 1 : 1 vermischt, dann wurde der pH-Wert mit Ammoniak bzw. Weinsäure auf einen Wert von 9 eingestellt. Es wurden die Farbstoffvorprodukte in den Kombinationen verwendet, die in Tabelle 1 aufgeführt sind.

Das erhaltene gebrauchsfertige Haarfärbemittel wurde auf eine Haarsträhne zu 90% ergrauten, nicht vorbehandelten Menschenhaars aufgebracht (Flotten-Gewichtsverhältnis: Gelmischung zu Haare = 2 zu 1) und mit einer Applicette gleichmäßig verteilt. Nach einer Einwirkzeit von 30 Minuten bei 32 °C wurde die Strähne mit lauwarmem Wasser ausgespült und danach im warmen Luftstrom (30°C bis 40°C) getrocknet. Die Färbungen sind der Tabelle 1 zu entnehmen.

### 3.0 Saure Spülung

Dann wurde jede gefärbte Haarsträhne aus Punkt 2.0 mit jeweils einer wässrigen auf einen pH-Wert von pH 3 eingestellten Lösung gespült und anschließend im warmen Luftstrom getrocknet. Die Färbung des Haars wurde erneut begutachtet und ist der Tabelle 1 zu entnehmen.

### 4.0 Alkalische Spülung

Dann wurde jede umgefärbte Haarsträhne aus Punkt 3.0 mit jeweils einer wässrigen auf einen pH-Wert von pH 9 eingestellten Lösung gespült und anschließend im warmen Luftstrom getrocknet. Die Färbung des Haars wurde erneut begutachtet und ist der Tabelle 1 zu entnehmen.

### Verbindungen der Komponente A (Tabelle 1):

| | |
|---|---|
| A1 | 1,2-Dihydro-1,3,4,6-tetramethyl-2-oxo-pyrimidinium-hydrogensulfat |

### Verbindungen der Komponente B (Tabelle 1):

| | |
|---|---|
| B1 | 4-Hydroxy-3-methoxybenzaldehyd (Vanillin) |
| B2 | 3-Ethoxy-4-hydroxybenzaldehyd (Ethylvanillin) |
| B3 | 3,5-Dimethoxy-4-hydroxybenzaldehyd |
| B4 | 4-Hydroxy-2-methoxybenzaldehyd |
| B5 | 4-Hydroxy-1-naphthaldehyd |
| B6 | 3,4-Dihydroxybenzaldehyd |
| B7 | 2,4-Dihydroxybenzaldehyd |

**Tabelle 1:**

| Komponente A (Gel 1) | Komponente B (Gel 2) | initiale Färbung | nach saurer Spülung | nach alkalischer Spülung |
|---|---|---|---|---|
| A1 | B1 | violett | olivbraun | violett |
| A1 | B2 | violett | olivbraun | violett |
| A1 | B3 | blauviolett | mittelbraun | blauviolett |
| A1 | B4 | rot | gelborange | rot |
| A1 | B5 | dunkelblau | beige | dunkelblau |
| A1 | B6 | violett | braungelb | violett |
| A1 | B7 | rot | gelb | rot |

### 3.0. UV/Vis-spektroskopische Vermessung der Färbelösungen bei verschiedenen pH-Werten

Es wurden 0,0015 mol der Komponente A1 (1,2-Dihydro-1,3,4,6-tetramethyl-2-oxopyrimidiniumhydrogensulfat) in 30 ml destilliertem Wasser gelöst. Anschließend wurden 0,0015 ml eines unter Punkt 2.0 beschriebenen aromatischen Aldehyds (Komponente B) in 30 ml destilliertem Wasser gelöst bzw. suspendiert. Beide Lösungen wurden vereinigt, und mit Ammoniaklösung wurde ein pH-Wert von 9 eingestellt. Anschließend wurde die Lösung für 30 Minuten bei ca. 30 °C aufbewahrt.

Die Lösung wurde mit verdünnter Salzsäure auf einen pH-Wert von 3 gebracht und nach geeigneter Verdünnung UV/Vis-spektroskopisch vermessen. Nach der Einstellung eines pH-Wertes von 9 mit verdünnter Natronlauge wurde die Lösung ein zweites Mal geeignet verdünnt und UV/Vis-spektroskopisch vermessen. Die UV/\/is-Spektren wurden im Wellenlängenbereich von 300 bis 700 nm aufgenommen. Die in Tabelle 2 aufgelisteten λₘₐₓ-Werte spiegeln die in Abhängigkeit vom pH-Wert auftretenden Farbverschiebungen wieder.

**Tabelle 2**

| Komponente A | Komponente B | λₘₐₓ (pH = 3) [300 - 700 nm] | λₘₐₓ (pH = 9) [300 - 700 nm] |
|---|---|---|---|
| A1 | B1 | 311; 427 | 317; 350; 542 |
| A1 | B3 | 306; 434 | 310; 363; 571 |
| A1 | B4 | 313; 440 | 319; 528 |
| A1 | B6 | 312; 428 | 316; 346; 536 |
| A1 | B7 | 310; 442 | 318; 538 |

## Patentansprüche

1. Verpackungselnheit (Kit), enthaltend
- in einem Container 1a ein Mittel, enthaltend in einem kosmetischen Träger mindestens eine CH-acide Verbindung, ausgewählt aus der Gruppe, die gebildet wird aus Verbindungen gemäß Formeln I und/oder deren Enaminformen, und aus Verbindungen der Formel (II), und Verbindungen der Formel (III) 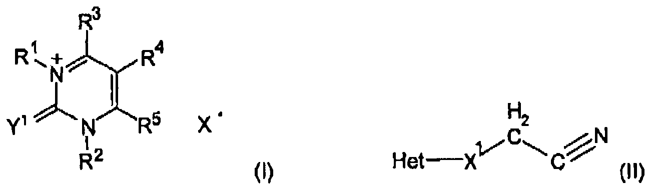 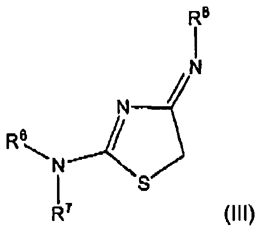 worin
• R¹ und R² stehen unabhängig voneinander für eine lineare oder cyclische C₁-C₆-Alkylgruppe, eine C₂-C₆-Alkonylgruppe, eine gegebenenfalls substituierte Arylgruppe, eine gegebenenfalls substituierte Heteroarylgruppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxy-C₁-C₆-alkylgruppe, eine Gruppe R^{I}R^{II}N-(CH₂)ₚ-, worin R^{I} und R^{II} stehen unabhängig voneinander für ein Wasserstoffatom, eine C₁-C₄-Alkylgruppe, eine C₁-C₄-Hydroxyalkylgruppe oder eine Aryl-C₁-C₆-alkylgruppe, wobei R^{I} und R^{II} gemeinsam mit dem Stickstoffatom einen 5-, 6- oder 7-gliedrigen Ring bilden können und p steht für eine Zahl 2, 3, 4, 5 oder 6,
• R³ und R⁵ stehen unabhängig voneinander für ein Wasserstoffatom oder eine C₁-C₆-Alkylgruppe, wobei mindestens einer der Reste R³ oder R⁵ eine C₁-C₆-Alkylgruppe bedeutet,
• R⁴ steht für ein Wasserstoffatom, eine C₁-C₆-Alkylgruppe, eine C₁-C₆-Hydroxyalkrylgruppe, eine C₂-C₆-Polyhydroxyalkylgruppe, eine C₁-C₆-Alkoxygruppe, eine C₁-C₆-Hydroxyalkoxygruppe, eine Gruppe R^{III}RI^{V}N-(CH₂)_{q}-, worin R^{III} und R^{IV} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und q steht für eine Zahl 1, 2, 3, 4, 5 oder 6, wobei der Rest R⁴ zusammen mit einem der Reste R³ oder R⁵ einen 5-oder 6-gliedrigen aromatischen Ring bilden kann, der gegebenenfalls mit einem Halogenatom, einer C₁-C₆-Alkylgruppe, einer C₁-C₆-Hydroxyalkylgruppe, einer C₂-C₆-Polyhydroxyalkylgruppe, einer C₁-C₆-Alkoxygruppe, einer C₁-C₆-Hydroxyalkoxygruppe, einer Nitrogruppe, einer Hydroxygruppe, einer Gruppe R^{V}R^{VI}N-(CH₂)₆-, worin R^{V} und R^{VI} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und s steht für eine Zahl 0, 1, 2, 3, 4, 5 oder 6 substituiert sein kann,
• Y¹ steht für ein Sauerstoffatom, ein Schwefelatom oder eine Gruppe NR^{VII}, worin R^{VII} steht für ein Wasserstoffatom, eine Arylgruppe, eine Heteroarylgruppe, eine C₁-C₆-Alkylgruppe oder eine C₁-C₆-Arylalkylgruppe,
• X⁻ steht für ein physiologisch verträgliches Anion.
• Het steht für einen gegebenenfalls substituierten Heteroaromaten,
• X¹ steht für eine direkte Bindung oder eine Carbonylgruppe.
• R⁶ und R⁷ bilden entweder zusammen mit dem Stickstoffatom einen gesättigten oder ungesättigten 5- oder 6-gliedrigen Ring oder stehen unabhängig voneinander für eine (C₁-C₆)-Alkylgruppe, eine (C₂-C₆)-Alkenylgruppe, eine Arylgruppe, eine Aryl-(C₁-C₆)-alkylgruppe, eine (C₂-C₆)-Hydroxyalkylgruppe, eine (C₂-C₆)-Polyhydroxyalkylgruppe oder eine Gruppe R^{I}R^{II}N-(CH₂)ₘ-, worin R^{I} und R^{II} stehen unabhängig voneinander für ein Wasserstoffatom, eine (C₁-C₆)-Alkylgruppe, eine (C₁-C₆)-Alkenylgruppe oder eine Aryl-C₁-C₆-alkylgruppe, wobei R^{I} und R^{II} gemeinsam mit dem Stickstoffatom einen 5-, 6- oder 7-gliedrigen Ring bilden können und m steht für eine Zahl 2, 3, 4, 5 oder 6, und
• R⁸ ein Wasserstoffatom, eine (C₁-C₆)-Alkylgruppe, eine (C₂-C₆)-Alkenylgruppe, eine Arylgruppe, eine Aryl-(C₁-C₆)-alkylgruppe, eine (C₂-C₆)-Hydroxyalkylgruppe, eine (C₂-C₆)-Polyhydroxyalkylgruppe oder eine Gruppe R^{III}R^{IV}N-(CH₂)ₙ-, worin R^{III} und R^{IV} stehen unabhängig voneinander für ein Wasserstoffatom, eine (C₁-C₆)-Alkylgruppe, eine (C₁-C₆)-Alkenylgruppe oder eine Aryl-C₁-C₆-alkylgruppe, wobei R^{III} und R^{IV} gemeinsam mit dem Stickstoffatom einen 5-, 6- oder 7-gliedrtgen Ring bilden können und n steht für eine Zahl 2, 3, 4, 5 oder 6,
- in einem Container 1b ein Mittel, enthaltend in einem kosmetischen Träger mindestens einen Aldehyd gemäß Formel (IV) 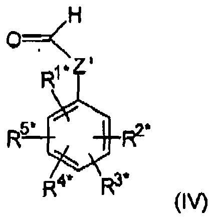 worin
• R^{1*}, R^{2*} und R^{3*} stehen unabhängig voneinander für ein Wasserstoffatom, ein Halogenatom, eine C₁-C₆-Alkylgruppe, eine Hydroxygruppe, eine C₁-C₆-Alkoxygruppe, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxygruppe, eine Sulfonsäuregruppe, eine Sulfonamidogruppe, eine Sulfonamidgruppe, Carbamoylgruppe, eine C₂-C₆-Acylgruppe oder eine Nitrogruppe,
• Z' steht für eine direkte Bindung oder eine Vinylengruppe,
• R^{4*} und R^{5*} stehen für ein Wasserstoffatom oder bilden gemeinsam, zusammen mit dem Restmolekül einen 5- oder 6-gliederigen aromatischen oder aliphatischen Ring.
- in einem Container 2 ein kosmetisches Mittel mit einem sauren pH-Wert und
- in einem Container 3 ein kosmetisches Mittel mit einem alkalischen pH-Wert.

2. Kit nach Anspruch 1, **dadurch gekennzeichnet, daß** die Verbindungen gemäß Formel I ausgewählt aus einer oder mehrerer Verbindungen der Gruppe von Salzen mit physiologisch verträglichem Gegenion X⁻, die gebildet wird aus Salzen des
1,2-Dihydro-1,3,4,6-tetramethyl-2-oxo-pyrimidiniums,
1,2-Dihydro-1,3-diethyl-4,B-dimethyl-2-oxo-pyrimidiniums,
1,2-Dihydro-1,3-dipropyl-4,6-dimethyl-2-oxo-pyrimidiniums,
1,2-Dihydro-1,3-di(2-hydroxyethyl)-4,6-dimethyl-2-oxo-pyrimidiniums,
1,2-Dihydro-1,3-diphenyl-4,8-dimethyl-2-oxo-pyrimidiniums,
1,2-Dihydro-1,3,4-trimethyl-2-oxo-pyrimidiniums,
1,2-Dihydro-1,3-diethyl-4-methyl-2-oxo-pyrimidiniums,
1,2-Dihydro-1,3-dipropyl-4-methyl-2-oxo-pyrimidiniums,
1,2-Dihydro-1,3-di(2-hydroxyethyl)-4-methyl-2-oxo-pyrimidiniums,
1,2-Dihydro-1,3-diphenyl-4-methyl-2-oxo-pyrimidiniums,
1-Allyl-1,2-dihydro-3,4,6-trimethyl-2-oxo-pyrimidiniums,
1,2-Dihydro-1-(2-hydroxyethyl)-3,4,6-trimethyl-2-oxo-pyrimidiniums,
1,2-Dihydro-1,3,4,6-tetramethyl-2-thioxo-pyrimidiniums,
1,2-Dihydro-1,3-diethyl-4,6-dimethyl-2-thioxo-pyrimidlnlums,
1,2-Dihydro-1,3-dipropyl-4,6-dimethyl-2-thioxo-pyrimidiniums,
1,2-Dihydro-1,3-di(2-hydroxyethyl)-4,6-dimethyl-2-thioxo-pyrimidiniums,
1,2-Dihydro-1,3-diphenyl-4,6-dimethyl-2-thioxo-pyrimidiniums,
1,2-Dihydro-1,3,4-trimethyl-2-thioxo-pyrimidiniums,
1,2.Dihydro-1,3-diethyl-4-methyl-2-thioxo-pyrimidiniums,
1,2-Dihydro-1,3-dipropyl-4-methyl-2-thioxo-pyrimidinlums,
1,2-Dihydro-1,3-di(2-hydroxyethyl)-4-methyl-2-thioxo-pyrimidiniums,
1,2-Dihydro-1,3-diphenyl-4-methyl-2-thioxo-pyrimidiniums,
1,2-Dihydro-3,4-dimethyl-2-oxo-chinazoliniums und
1,2-Dihydro-3,4-dimethyl-2-thioxo-chinazoliniums.

3. Kit nach einem der Ansprüche 1 oder 2, **dadurch gekennzeichnet, daß** die Verbindungen gemäß Formel (II) ausgewählt werden aus solchen, in denen sich der Rest Het gemäß Formel II ableitet von einem der Heteroaromaten Furan, Thiophen, Pyrrol, Isoxazol, Isothiazol, Imidazol, Oxazol, Thiazol, Pyridin, Pyridazin, Pyrimidin, Pyrazin, 1,2,3-Triazin, 1,2,4-Triazin, 1,3,5-Triazin, Benzopyrrol, Benzofuren, Benzothiophen, Benzimidazol, Benzoxazol, Indazol, Benzoisoxazol, Benzoisothiazol, Indol, Chinolin, Isochinolin, Cinnolin, Phthalazin, Chinazolin, Chinoxalin, Acridin, Benzochinolin, Benzoisochinolin, Phenazin, Benzocinnolin, Benzochinazolin, Benzochinoxalin, Phenoxazin, Phenothiazin, Nephthyridin, Phenanthrolin, Indolizin, Chinolizin, Carbolin, Purin, Pteridin und Cumarin, wobei die vorgenannten Heteroaromaten mit mindestens einer Gruppe ausgewählt aus einem Halogenatom, einer Nitrogruppe, einer Thiogruppe, einer Thio-(C₁-C₆)-alkylgruppe, einer Heteroarylgruppe, einer Arylgruppe, einer (C₁-C₆)-Alkylgruppe, einer (C₁-C₆)-Alkoxygruppe, einer Hadroxygruppe, einer (C₂-C₆)-Hydroxyalkylgruppe, einer (C₂-C₆)-Polyhydroxyalkylgruppe, einer (C₁-C₆)-Alkoxyl-(C₁-C₆)-alkylgruppe, einer Aryl-(C₁-C₆)-alkylgruppe, einer Aminogruppe, einer (C₁-C₆)-Monoalkylaminogruppe, einer (C₁-C₆)-Dialkylaminogruppe, eine Dialkylaminoalkylgruppe -(CH₂)ₙ-NR'R", worin n eine ganze Zahl von 2 und 6 ist und R' und R" unabhängig voneinander eine lineare oder verzweigte Alkylgruppe bedeutet, welche gegebenenfalls zusammen einen Ring bilden können, substituiert sein können.

4. Kit nach einem der Ansprüche 1 bis 3, **dadurch gekennzeichnet, daß** die Verbindungen gemäß Formel II ausgewählt werden aus mindestens einem Vertreter der Gruppe bestehend aus 2-(2-Furoyl)-acetonitril, 2-(5-Brom-2-furoyl)-acetonitril, 2-(5-Methyl-2-trifluormethyl-3-furoyl)-acetonitril, 3-(2,5-Dimethyl-3-furyl)-3-oxopropanitril, 2-(2-Thenoyl)-acetonitril, 2-(3-Thenoyl)-acetonitril, 2-(5-Fluor-2-thenoyl)-acetonitril, 2-(5-Chlor-2-thenoyl)-acetonitril, 2-(5-Brom-2-thenoyl)-acetonitril, 2-(5-Methyl-2-thenoyl)-acetonitril, 2-(2,5-Dimethylpyrrol-3-oyl)-acetonitril, 2-(1,2,5-Trimethylpyrrol-3-oyl)-acetonitril, 1H-Benzimidazol-2-ylacetonitril, 1H-Benzothiazol-2-ylacetonitril, 2-(Pyrid-2-yl)-acetonitril, 2,6-Bis(cyanmethyl)-pyridin, 2-(Indol-3-oyl)-acetonitril, 2-(2-Methyl-indol-3-oyl)-acetonitril, 8-Cyanacetyl-7-methoxy-4-methylcumarin, 2-(2-Isopropyl-5,6-benzochinolini4-oyl)-acetonitril, 2-(2-Phenyl-5,6-benzochinolin-4-oyl)-acetonitril, 2-(Chinoxalin-2-yl)-acetonitril, 2-(Cumaron-2-yl)-acetonitril, 6,7-Dichlor-5-(cyanoacetyl)-2,3-dihydro-1-benzofuran-2-carbonsäure-tert.-butylester, 2-(6-Hydroxy-4,7-dimethoxy-1-benzofuran-5-oyl)-acetonitril und 2-(1-Phenyl-1,4-dihydrothiochromeno[4,3-c]pyrazol-3-oyl)-acetonitril.

5. Kit nach einem der Ansprüche 1 bis 4, **dadurch gekennzeichnet, daß** die Verbindungen der Formel (III) aus den Vertretern ausgewählt werden, bei denen die Reste R⁶ und R⁷ gemäß Formel (III) zusammen mit dem Stickstoffatom einen gesättigten 5- oder 6-gliedrigen Ring bilden.

6. kit nach einem der Ansprüche 1 bis 5, **dadurch gekennzeichnet, daß** die Verbindungen der Formeln (I), (II) und (III) ausgewählt werden, aus mindestens einem Vertreter aus
| | |
|---|---|
| Salzen des | 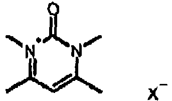 |
| 1,2-Dihydro-1,3,4,6-tetramethyl-2-oxopyrimidiniums | |
| Salzen des | 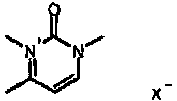 |
| 1,2-Dihydro-1,3,4-trimethyl-2-oxopyrimidiniums | |
| Salzen des | 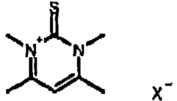 |
| 1,2-Dihydro-1,3,4,6-tetramethyl-2-thioxopyrimidiniums | |
| Salzen des | 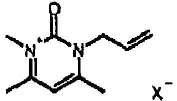 |
| 1-Allyl-1,2-dihydro-3,4,6-trimethyl-2-oxopyrimidiniums | |
| Salzen des | 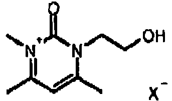 |
| 1,2-Dihydro-1-(2-hydroxyethyl)-3,4,8-trimethyl-2-oxopyrimidiniums | |
| | 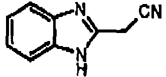 |
| 2-(Cyanomethyl)benzimidazol | |
| | 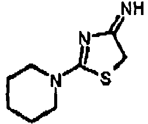 |
| 4,5-Dihydro-4-imino-2-(1-piperidinyl)-thiazol | |
| | 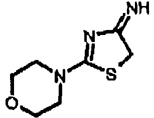 |
| 4,5-Dihydro-4-imino-2-(4-morpholinyl)-thiazol | |
| | 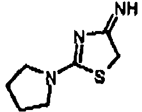 |
| 4,5-Dihydro-4-imino-2-(1-pyrrolidinyl)-thiazol | |
wobei die Salze der vorgenannten Verbindungen X⁻ als ein physiologisch verträgliches Gegenion enthalten.

7. Kit nach einem der Ansprüche 1 bis 6, **dadurch gekennzeichnet, daß** die Verbindungen der Formel (IV) ausgewählt werden aus mindestens einer Verbindung der Gruppe 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldahyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzoldehyd, 4-Dimethylamino-2-methoxybenzaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldahyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxyberizaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzeldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl.benzaldehyd, 2,4-Dihydroxy-6-methyl-benzaldehyd, 2,4-Dihydroxy-3-methoxy-benzaldehyd, 2,4-Dihydroxy-5-methoxy-benzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzalde-hyd, 4-Diethylaminobenzaldehyd, 4-Dimethylamino-2-hydroxybenzaldehyd, 4-Pyrrolidinobenzaldehyd, 4-Morpholinobenzaldehyd, 2-Morpholinobenzaldahyd, 4-Piperidinobenzaldehyd, 3,5-Dichlor-4-hydroxybenzaldehyd, 4-Hydroxy-3,5-diiod-benzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldahyd, 4-Hydroxy-3-lod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 3-Hydroxy-4-nitrobenzaldehyd, 2-Hydroxy-3-methoxy-5-nitrobenzaldehyd, 5-Nitrovanillin, 2,5-Dinitrosalicylaldehyd, 5-Brom-3-nitrosalicylaldehyd, 2-Dimethylamlnobenzaldehyd, 2-Chlor-4-dimethylamlnobenzaldehyd, 4-Dimethylamlno-2-methylbenzaldehyd, 4-Diethylamino-zimtaldehyd, 4-Dibutylamino-benzaldehyd, 3-Allyl-4-hydroxybenzaldehyd, 3-Allyl-4-hydroxy-5-methoxybenzaldehyd, 3-Allyl-4-hydroxy-5-methylbenzaldehyd, 3-Allyl-5-brom-4-hydroxybenzaldehyd, 3,5-Diallyl-4-hydroxybenzaldehyd, 3-Allyl-4-hydroxy-5-formylbenzaldehyd (5-Allyl-4-hydroxyisophthalaldehyd) und Piperonal.

8. Kit nach einem der Ansprüche 1 bis 7, **dadurch gekennzeichnet, daß** das kosmetische Mittel mit einem sauren pH-Wert einen pH-Wert von pH 2 bis pH 6 besitzt.

9. Kit nach einem der Ansprüche 1 bis 8, **dadurch gekennzeichnet, daß** das kosmetische Mittel mit einem sauren pH-Wert keine farbgebenden Komponenten enthält.

10. Kit nach einem der Ansprüche 1 bis 9, **dadurch gekennzeichnet, daß** das kosmetische Mittel mit einem alkalischen pH-Wert einen pH-Wert von pH 8 bis pH 11 besitzt.

11. Kit nach einem der Ansprüche 1 bis 10, **dadurch gekennzeichnet, daß** das kosmetische Mittel mit einem alkalischen pH-Wert keine farbgebenden Komponenten enthält.

12. Verfahren zur reversiblen Umfärbung keratinhaltiger Fasern, insbesondere menschlicher Haare, welche zuvor mit einem Färbemittel, enthaltend in einem kosmetischen Träger eine Kombination aus Komponente
(A) mindestens einer Verbindung der Formel (I) und/oder (II) und/oder (III) 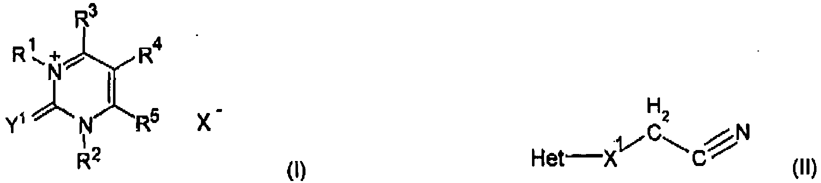 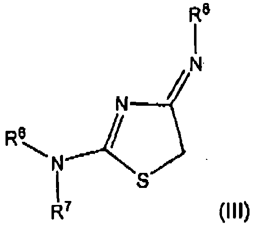 worin R¹, R², R³, R⁴, R⁵, R⁶, R⁷, R⁸, Het, X¹ und Y¹ wie im ersten Anspruch definiert sind
mit Komponente (B) mindestens einer Verbindung gemäß Formel (IV) 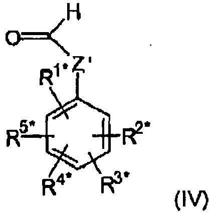 worin R^{1*}, R^{2*}, R^{3*}, R^{4*}, R^{5*} und Z' wie im ersten Anspruch definiert sind,
gefärbt wurden, und
(a) die gefärbten keratinhaltigen Fasem mit einem kosmetischen Mittel mit einem sauren pH-Wert umgefärbt werden und
(b) gegebenenfalls nach einem Zeitraum von bis zu 4 Wochen mit einem kosmetischen Mittel mit einem alkalischen pH-Wert wieder umgefärbt werden.

13. Verfahren nach Anspruch 12, **dadurch gekennzeichnet, daß** das kosmetische Mittel mit einem sauren pH-Wert nicht vom Haar gespült wird.

14. Verfahren nach einem der Ansprüche 12 oder 13, **dadurch gekennzeichnet, daß** das kosmetische Mittel mit einem alkalischen pH-Wert nicht vom Haar gespült wird.

15. Verfahren nach einem der Ansprüche 12 bis 14, **dadurch gekennzeichnet, daß** das kosmetische Mittel mit einem sauren pH-Wert einen pH-Wert von pH 2 bis pH 6 besitzt.

16. Verfahren nach einem der Ansprüche 12 bis 15, **dadurch gekennzeichnet, daß** das kosmetische Mittel mit einem sauren pH-Wert keine farbgebenden Komponenten enthält.

17. Verfahren nach einem der Ansprüche 12 bis 16, **dadurch gekennzeichnet, daß** das kosmetische Mittel mit einem alkalischen pH-Wert einen pH-Wert von pH 8 bis pH 11 besitzt.

18. Verfahren nach einem der Ansprüche 12 bis 17, **dadurch gekennzeichnet, daß** das kosmetische Mittel mit einem alkalischen pH-Wert keine farbgebenden Komponenten enthält.

19. Verwendung eines kosmetischen Mittels mit einem sauren pH-Wert zur Farbänderung keratinhaltiger Fasern, insbesondere menschlicher Haare, die mit einem Färbemittel, enthaltend In einem kosmetischen Träger eine Kombination aus Komponente
(A) mindestens einer Verbindung der Formel (I) und/oder (II) und/oder (III) 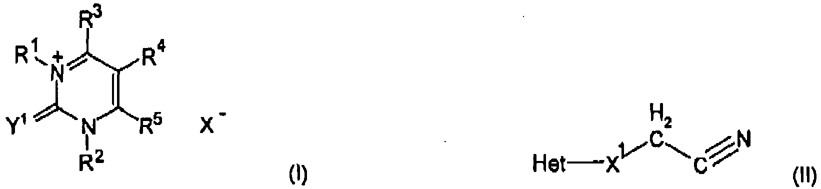 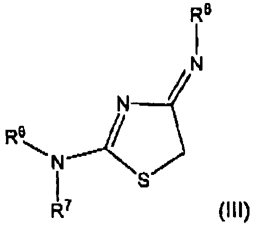 worin R¹, R², R³, R⁴, R⁵, R⁶, R⁷, R⁸, Het, X¹ und Y¹ wie im ersten Anspruch definiert sind
mit Komponente (B) mindestens einer Verbindung gemäß Formel (IV) 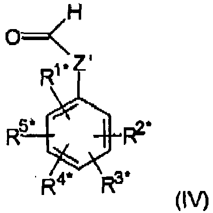 worin R^{1*}, R^{2*}, R^{3*}, R^{4*}, R^{5*} und Z' wie Im ersten Anspruch definiert sind,
gefärbt wurden.

20. Verwendung eines kosmetischen Mittels mit einem alkalischen pH-Wert zur Wiederherstellung einer Färbung keratinhaltiger Fasern, insbesondere menschlicher Haare, die zunächst mit einem Färbemittel, enthaltend in einem kosmetischen Träger eine Kombination aus Komponente
(A) mindestens einer Verbindung der Formel (I) und/oder (II) und/oder (III) 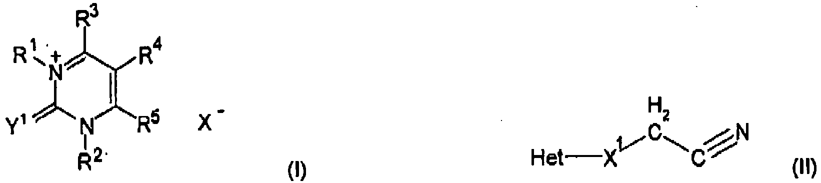 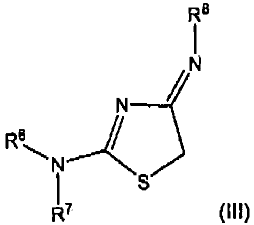 worin R¹, R², R³, R⁴, R⁵, R⁶, R⁷, R⁸, Het, X¹ und Y¹ wie im ersten Anspruch definiert sind
mit Komponente (B) mindestens einer Verbindung gemäß Formel (IV) 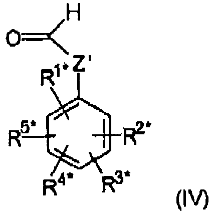 worin R^{1*}, R^{2*}, R^{3*}, R^{4*}, R^{5*} und Z' wie im ersten Anspruch definiert sind,
gefärbt wurden und dann mit einem kosmetischen Mittel mit saurem pH-Wert einer Farbveränderung unterworfen wurden.

## Claims

1. Packaging unit, comprising
- in a container 1a an agent, comprising in a cosmetic carrier at least one CH-acidic compound, selected from the group consisting of compounds according to Formula (I) and/or their enamine forms, and of compounds of Formula (II), and compounds of Formula (III) 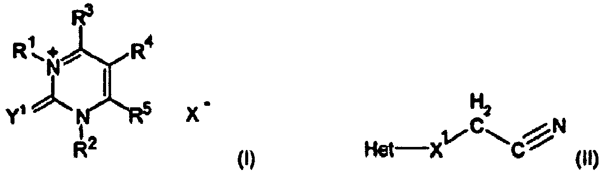 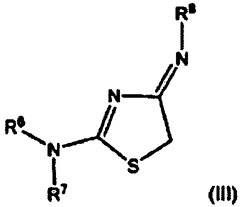 in which
• R¹ and R² independently of one another stand for a linear or cyclic C₁-C₆ alkyl group, a C₂-C₆ alkenyl group, an optionally substituted aryl group, an optionally substituted heteroaryl group, an aryl C₁-C₆ alkyl group, a C₁-C₆ hydroxyalkyl group, a C₂-C₆ polyhydroxyalkyl group, a C₁-C₆ alkoxy C₁-C₆ alkyl group, a R^{I}R^{II}N-(CH₂)ₚ- group, in which R^{I} and R^{II} stand independently of one another for a hydrogen atom, a C₁-C₄ alkyl group, a C₁-C₄ hydroxyalkyl group or an aryl C₁-C₆ alkyl group, wherein R^{I} and R^{II} together with the nitrogen atom can form a 5-, 6- or 7-membered ring and p stands for a number 2, 3, 4, 5 or 6,
• R³ and R⁵ independently of one another stand for a hydrogen atom or a C₁-C₆ alkyl group, wherein at least one of the groups R³ or R⁵ means a C₁-C₆ alkyl group,
• R⁴ stands for a hydrogen atom, a C₁-C₆ alkyl group, a C₁-C₆ hydroxyalkyl group, a C₂-C₆ polyhydroxyalkyl group, a C₁-C₆ alkoxy group, a C₁-C₆ hydroxyalkoxy group, a R^{III}R^{IV}N-(CH₂)_{q}- group, in which R^{III} and R^{IV} stand independently of one another for a hydrogen atom, a C₁-C₆ alkyl group, a C₁-C₆ hydroxyalkyl group or an aryl C₁-C₆ alkyl group, and q stands for a number 1, 2, 3, 4, 5 or 6, wherein the R⁴ residue together with one of the residues R³ or R⁵ can form a 5- or 6-membered aromatic ring that can be optionally substituted with a halide atom, a C₁-C₆ alkyl group, a C₁-C₆ hydroxyalkyl group, a C₂-C₆ polyhydroxyalkyl group, a C₁-C₆ alkoxy group, a C₁-C₆ hydroxyalkoxy group, a nitro group, a hydroxyl group, a R^{V}R^{VI}N-(CH₂)ₛ- group, in which R^{V} and R^{VI} stand independently of one another for a hydrogen atom, a C₁-C₆ alkyl group, a C₁-C₆ hydroxyalkyl group or an aryl C₁-C₆ alkyl group, and s stands for a number 0, 1, 2, 3, 4, 5 or 6,
• Y¹ stands for an oxygen atom, a sulfur atom or a group NR^{VII}, in which R^{VII} stands for a hydrogen atom, an aryl group, a heteroaryl group, a C₁-C₆ alkyl group or a C₁-C₆ arylalkyl group,
• X- stands for a physiologically acceptable anion,
• Het stands for an optionally substituted heteroaromatic group,
• X¹ stands for a direct bond or a carbonyl group.
• R⁶ and R⁷ either form together with the nitrogen atom a saturated or unsaturated 5- or 6-membered ring or stand independently of one another for a C₁-C₆ alkyl group, a C₂-C₆ alkenyl group, an aryl group, an aryl C₁-C₆ alkyl group, a C₂-C₆ hydroxyalkyl group, a C₂-C₆ polyhydroxyalkyl group or a R^{I}R^{II}N-(CH₂)ₘ- group, in which R^{I} and R^{II} stand independently of one another for a hydrogen atom, a C₁-C₆ alkyl group, a C₁-C₆ alkenyl group or an aryl C₁-C₆ alkyl group, wherein R^{I} and R^{II} together with the nitrogen atom can form a 5-, 6- or 7-membered ring and m stands for a number 2, 3, 4, 5 or 6, and
• R⁸ stands for a C₁-C₆ alkyl group, a C₂-C₆ alkenyl group, an aryl group, an aryl C₁-C₆ alkyl group, a C₂-C₆ hydroxyalkyl group, a C₂-C₆ polyhydroxyalkyl group or a R^{III}R^{IV}N-(CH₂)ₙ- group, wherein R^{III} and R^{IV} stand independently of one another for a hydrogen atom, a C₁-C₆ alkyl group, a C₁-C₆ alkenyl group or an aryl C₁-C₆ alkyl group, wherein R^{III} and R^{IV} together with the nitrogen atom can form a 5-, 6- or 7 membered ring and n stands for a number 2, 3, 4, 5 or 6,
- in a container 1b an agent, comprising in a cosmetic carrier at least one aldehyde according to Formula (IV) 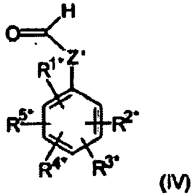 in which
• R^{1*}, R^{2*} und R^{3*} independently of each other stand for a hydrogen atom, a halogen atom, a C₁-C₆ alkyl group, a hydroxyl group, a C₁-C₆ alkoxy group, a C₁-C₆ dialkylamino group, a di(C₂-C₆ hydroxyalkyl)amino group, a di(C₁-C₆ alkoxy C₁-C₆ alkyl)amino group, a C₁-C₆ hydroxyalkyloxy group, a sulfonyl group, a carboxyl group, a sulfonic acid group, a sulfonamido group, a sulfonamide group, carbamoyl group, a C₂-C₆ acyl group or a nitro group,
• Z' stands for a direct bond or a vinylene group,
• R^{4*} und R^{5*} stand for a hydrogen atom or together, with the rest of the molecule, form a 5- or 6-membered aromatic or aliphatic ring.
- in a container 2 a cosmetic agent with an acidic pH and
- in a container 3 a cosmetic agent with an alkaline pH.

2. Kit according to claim 1, **characterised in that** the compounds according to Formula I are selected from one or a plurality of compounds of the group of salts with a physiologically acceptable counter ion X⁻, which is formed from salts of
1,2-Dihydro-1,3,4,6-tetramethyl-2-oxo-pyrimidinium,
1,2-Dihydro-1,3-diethyl-4,6-dimethyl-2-oxo-pyrimidinium,
1,2-Dihydro-1,3-dipropyl-4,6-dimethyl-2-oxo-pyrimidinium,
1,2-Dihydro-1,3-di(2-hydroxyethyl)-4,6-dimethyl-2-oxo-pyrimidinium,
1,2-Dihydro-1,3-diphenyl-4,6-dimethyl-2-oxo-pyrimidinium,
1,2-Dihydro-1,3,4-trimethyl-2-oxo-pyrimidinium,
1,2-Dihydro-1,3-diethyl-4-methyl-2-oxo-pyrimidinium,
1,2-Dihydro-1,3-dipropyl-4-methyl-2-oxo-pyrimidinium,
1,2-Dihydro-1,3-di(2-hydroxyethyl)-4-methyl-2-oxo-pyrimidinium,
1,2-Dihydro-1,3-diphenyl-4-methyl-2-oxo-pyrimidinium,
1-Allyl-1,2-dihydro-3,4,6-trimethyl-2-oxo-pyrimidinium,
1,2-Dihydro-1-(2-hydroxyethyl)-3,4,6-trimethyl-2-oxo-pyrimidinium,
1,2-Dihydro-1,3,4,6-tetramethyl-2-thioxo-pyrimidinium,
1,2-Dihydro-1,3-diethyl-4,6-dimethyl-2-thioxo-pyrimidinium,
1,2-Dihydro-1,3-dipropyl-4,6-dimethyl-2-thioxo-pyrimidinium,
1,2-Dihydro-1,3-di(2-hydroxyethyl)-4,6-dimethyl-2-thioxo-pyrimidinium,
1,2-Dihydro-1,3-diphenyl-4,6-dimethyl-2-thioxo-pyrimidinium,
1,2-Dihydro-1,3,4-trimethyl-2-thioxo-pyrimidinium,
1,2-Dihydro-1,3-diethyl-4-methyl-2-thioxo-pyrimidinium,
1,2-Dihydro-1,3-dipropyl-4-methyl-2-thioxo-pyrimidinium,
1,2-Dihydro-1,3-di(2-hydroxyethyl)-4-methyl-2-thioxo-pyrimidinium,
1,2-Dihydro-1,3-diphenyl-4-methyl-2-thioxo-pyrimidinium,
1,2-Dihydro-3,4-dimethyl-2-oxo-quinazolinium and
1,2-Dihydro-3,4-dimethyl-2-thioxo-quinazolinium.

3. Kit according to one of claims 1 or 2, **characterised in that** the compounds according to Formula (II) are selected from those, in which the Het residue according to Formula II derives from one of the heteroaromatics furan, thiophene, pyrrole, isoxazole, isothiazole, imidazole, oxazole, thiazole, pyridine, pyridazine, pyrimidine, pyrazine, 1,2,3-triazine, 1,2,4-triazine, 1,3,5-triazine, benzopyrrole, benzofuran, benzothiophene, benzimidazole, benzoxazole, indazole, benzoisoxazole, benzoisothiazole, indole, quinoline, isoquinoline, cinnoline, phthalazine, quinazoline, quinoxaline, acridine, benzoquinoline, benzoisoquinoline, phenazine, benzocinnoline, benzoquinazoline, benzoquinoxaline, phenoxazine, phenothiazine, nephthyridine, phenanthroline, indolizine, quinolizine, carboline, purine, pteridine and coumarin, wherein the cited heteroaromatics can be substituted by at least one group selected from the group consisting of a halogen atom, a nitro group, a thio group, a thio-(C₁-C₆) alkyl group, a heteroaryl group, an aryl group, a (C₁-C₆) alkyl group, a (C₁-C₆) alkoxy group, a hydroxyl group, a (C₂-C₆) hydroxyalkyl group, a (C₂-C₆) polyhydroxyalkyl group, a (C₁-C₆) alkoxy (C₁-C₆) alkyl group, an aryl (C₁-C₆) alkyl group, an amino group, a (C₁-C₆) monoalkylamino group, a (C₁-C₆) dialkylamino group, a dialkylaminoalkyl group -(CH₂)ₙ-NR'R"; wherein n is a whole number from 2 to 6 and each of R' and R" is independently a linear or branched alkyl group which together can optionally form a ring.

4. Kit according to one of claims 1 to 3, **characterised in that** the compounds according to Formula II are selected from at least one representative of the group consisting of 2-(2-furoyl)acetonitrile, 2-(5-bromo-2-furoyl)acetonitrile, 2-(5-methyl-2-trifluoromethyl-3-furoyl)acetonitrile, 3-(2,5-dimethyl-3-furyl)-3-oxopropanitrile, 2-(2-thenoyl)acetonitrile, 2-(3-thenoyl)acetonitrile, 2-(5-fluoro-2-thenoyl)acetonitrile, 2-(5-chloro-2-thenoyl)acetonitrile, 2-(5-bromo-2-thenoyl)acetonitrile, 2-(5-methyl-2-thenoyl)acetonitrile, 2-(2,5-dimethylpyrrol-3-oyl)acetonitrile, 2-(1,2,5-trimethylpyrrol-3-oyl)acetonitrile, 1*H*-benzimidazol-2-ylacetonitrile, 1*H*-benzothiazol-2-ylacetonitrile, 2-(pyrid-2-yl)acetonitrile, 2,6-bis(cyanomethyl)pyridine, 2-(indol-3-oyl)acetonitrile, 2-(2-methylindol-3-oyl)acetonitrile, 8-cyanoacetyl-7-methoxy-4-methylcoumarin, 2-(2-isopropyl-5,6-benzoquinolin-4-oyl)acetonitrile, 2-(2-phenyl-5,6-benzoquinolin-4-oyl)acetonitrile, 2-(quinoxalin-2-yl)acetonitrile, 2-(coumaron-2-yl)acetonitrile, t-butyl 6,7-dichloro-5-(cyanoacetyl)-2,3-dihydro-1-benzofuran-2-carboxylate, 2-(6-hydroxy-4,7-dimethoxy-1-benzofuran-5-oyl)acetonitrile and 2-(1-phenyl-1,4-dihydrothiochromeno[4,3-c]pyrazol-3-oyl)acetonitrile.

5. Kit according to one of claims 1 to 4, **characterised in that** the compounds of the Formula (III) are selected from the representatives, in which the residues R⁶ and R⁷ according to the Formula (III) together with the nitrogen atom form a saturated 5- or 6-membered ring.

6. Kit according to one of claims 1 to 5, **characterised in that** the compounds of Formulas (I), (II) and (III) are selected from at least one representative of
| | |
|---|---|
| Salts of 1,2-dihydro-1,3,4,6-tetramethyl-2-oxopyrimidinium | 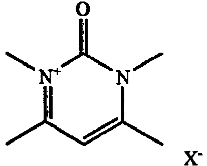 |
| Salts of 1,2-dihydro-1,3,4-trimethyl-2-oxopyrimidinium | 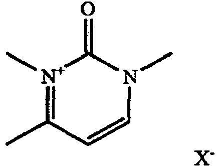 |
| Salts of 1,2-dihydro-1,3,4,6-tetramethyl-2-thioxopyrimidinium | 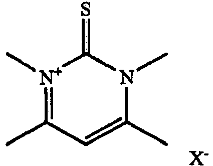 |
| Salts of 1-allyl-1,2-dihydro-3,4,6-trimethyl-2-oxopyrimidinium | 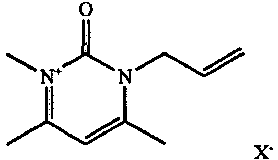 |
| Salts of 1,2-dihydro-1-(2-hydroxyethyl)-3,4,6-trimethyl-2-oxopyrimidinium | 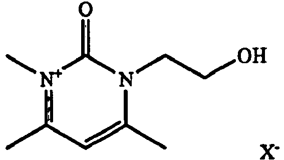 |
| 2-(Cyanomethyl)benzimidazole | 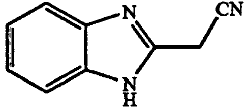 |
| 4,5-Dihydro-4-imino-2-(1-piperidinyl)thiazole | 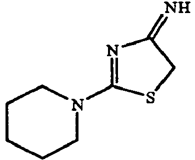 |
| 4,5-Dihydro-4-imino-2-(4-morpholinyl)thiazole | 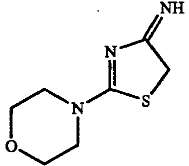 |
| 4,5-Dihydro-4-imino-2-(pyrrolidinyl)thiazole | 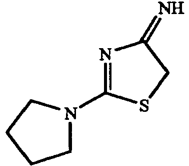 |
wherein the salts of the cited compounds comprise X- as a physiologically acceptable counter ion.

7. Kit according to one of claims 1 to 6, **characterised in that** the compounds of the Formula (IV) are selected from at least one compound of the group 4-hydroxy-3-methoxybenzaldehyde, 3,5-dimethoxy-4-hydroxybenzaldehyde, 4-hydroxy-1-naphthaldehyde, 4-hydroxy-2-methoxybenzaldehyde, 3,4-dihydroxy-5-methoxybenzaldehyde, 3,4,5-trihydroxybenzaldehyde, 3,5-dibromo-4-hydroxybenzaldehyde, 4-hydroxy-3-nitrobenzaldehyde, 3-bromo-4-hydroxybenzaldehyde, 4-hydroxy-3-methylbenzaldehyde, 3,5-dimethyl-4-hydroxybenzaldehyde, 5-bromo-4-hydroxy-3-methoxybenzaldehyde, 4-diethylamino-2-hydroxybenzaldehyde, 4-dimethylamino-2-methoxybenzaidehyde, 2-methoxybenzaldehyde, 3-methoxybenzaldehyde, 4-methoxybenzaldehyde, 2-ethoxybenzaldehyde, 3-ethoxybenzaldehyde, 4-ethoxybenzaldehyde, 4-hydroxy-2,3-dimethoxybenzaldehyde, 4-hydroxy-2,5-dimethoxybenzaldehyde, 4-hydroxy-2,6-dimethoxybenzaldehyde, 4-hydroxy-2-methylbenzaldehyde, 4-hydroxy-2,3-dimethylbenzaldehyde, 4-hydroxy-2,5-dimethylbenzaldehyde, 4-hydroxy-2,6-dimethylbenzaldehyde, 3,5-diethoxy-4-hydroxybenzaldehyde, 2,6-diethoxy-4-hydroxybenzaldehyde, 3-hydroxy-4-methoxybenzaldehyde, 2-hydroxy-4-methoxybenzaldehyde, 2-ethoxy-4-hydroxybenzaldehyde, 3-ethoxy-4-hydroxybenzaldehyde, 4-ethoxy-2-hydroxybenzaldehyde, 4-ethoxy-3-hydroxybenzaldehyde, 2,3-dimethoxybenzaldehyde, 2,4-dimethoxybenzaldehyde, 2,5-dimethoxybenzaldehyde, 2,6-dimethoxybenzaldehyde, 3,4-dimethoxybenzaidehyde, 3,5-dimethoxybenzaldehyde, 2,3,4-trimethoxybenzaldehyde, 2,3,5-trimethoxybenzaldehyde, 2,3,6-trimethoxybenzaldehyde, 2,4,6-trimethoxybenzaldehyde, 2,4,5-trimethoxybenzaidehyde, 2,5,6-trimethoxybenzaldehyde, 2-hydroxybenzaldehyde, 3-hydroxybenzaldehyde, 4-hydroxybenzaldehyde, 2,3-dihydroxybenzaldehyde, 2,4-dihydroxy-benzaldehyde, 2,4-dihydroxy-3-methylbenzaldehyde, 2,4-dihydroxy-5-methylbenzaldehyde, 2,4-dihydroxy-6-methylbenzaldehyde, 2,4-dihydroxy-3-methoxybenzaldehyde, 2,4-dihydroxy-5-methoxybenzaldehyde, 2,4-dihydroxy-6-methoxybenzaldehyde, 2,5-dihydroxybenzaldehyde, 2,6-dihydroxybenzaldehyde, 3,4-dihydroxybenzaldehyde, 3,4-dihydroxy-2-methylbenzaldehyde, 3,4-dihydroxy-5-methylbenzaidehyde, 3,4-dihydroxy-6-methylbenzaldehyde, 3,4-dihydroxy-2-methoxybenzaidehyde, 3,5-dihydroxybenzaldehyde, 2,3,4-trihydroxybenzaldehyde, 2,3,5-trihydroxybenzaldehyde, 2,3,6-trihydroxybenzaldehyde, 2,4,6-trihydroxybenzaldehyde, 2,4,5-trihydroxybenzaldehyde, 2,5,6-trihydroxybenzaldehyde, 4-dimethylaminobenzaldehyde, 4-diethylaminobenzaldehyde, 4-dimethylamino-2-hydroxybenzaldehyde, 4-pyrrolidinobenzaldehyde, 4-morpholinobenzaldehyde, 2-morpholinobenzaldehyde, 4-piperidinobenzaldehyde, 3,5-dichloro-4-hydroxybenzaldehyde, 4-hydroxy-3,5-diiodobenzaldehyde, 3-chloro-4-hydroxybenzaldehyde, 5-chloro-3,4-dihydroxybenzaldehyde, 5-bromo-3,4-dihydroxybenzaldehyde, 3-chloro-4-hydroxy-5-methoxybenzaldehyde, 4-hydroxy-3-iodo-5-methoxybenzaidehyde, 2-methoxy-1-naphthaldehyde, 4-methoxy-1-naphthaldehyde, 2-hydroxy-1-naphthaldehyde, 2,4-dihydroxy-1-naphthaldehyde, 4-hydroxy-3-methoxy-1-naphthaldehyde, 2-hydroxy-4-methoxy-1-naphthaldehyde, 3-hydroxy-4-methoxy-1-naphthaldehyde, 2,4-dimethoxy-1-naphthaldehyde, 3,4-dimethoxy-1-naphthaldehyde, 4-dimethylamino-1-naphthaldehyde, 3-hydroxy-4-nitrobenzaldehyde, 2-hydroxy-3-methoxy-5-nitrobenzaldehyde, 5-nitrovanillin, 2,5-dinitrosalicylaldehyde, 5-bromo-3-nitrosalicylaldehyde, 2-dimethylaminobenzaldehyde, 2-chloro-4-dimethylaminobenzaldehyde, 4-dimethylamino-2-methylbenzaldehyde, 4-diethylaminocinnamaidehyde, 4-dibutylaminobenzaldehyde, 3-allyl-4-hydroxybenzaldehyde, 3-allyl-4-hydroxy-5-methoxybenzaldehyde, 3-allyl-4-hydroxy-5-methylbenzaidehyde, 3-allyl-5-bromo-4-hydroxybenzaldehyde, 3,5-diallyl-4-hydroxybenzaldehyde, 3-allyl-4-hydroxy-5-formylbenzaldehyde (5-allyl-4-hydroxy-isophthalaldehyde) and piperonal.

8. Kit according to one of claims 1 to 7, **characterised in that** the cosmetic agent with an acidic pH has a pH of pH 2 to pH 6.

9. Kit according to one of claims 1 to 8, **characterised in that** the cosmetic agent with an acidic pH does not comprise a coloring component.

10. Kit according to one of claims 1 to 9, **characterised in that** the cosmetic agent with an alkaline pH has a pH of pH 8 to pH 11.

11. Kit according to one of claims 1 to 10, **characterised in that** the cosmetic agent with an alkaline pH does not comprise a coloring component.

12. Method for reversibly re-dyeing keratin-containing fibres, in particular human hair, which had been previously dyed with a coloring agent, comprising in a cosmetic carrier a combination of components
(A) at least one compound of the Formula (I) and/or (II) and/or (III) 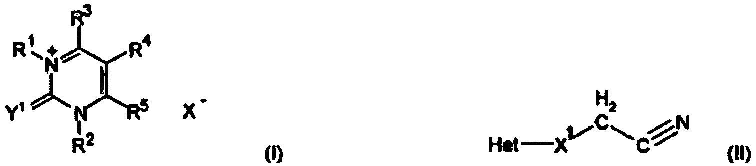 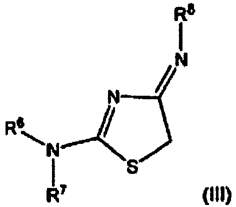 in which R¹, R², R³, R⁴, R⁵, R⁶, R⁷, R⁸, Het, X¹ and Y¹ are as defined in the first Claim
with component (B) at least one compound according to Formula (IV) 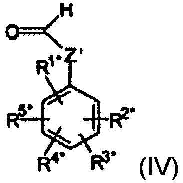 in which R^{1*}, R^{2*}, R^{3*}, R^{4*}, R^{5*} and Z' are as defined in the first Claim, and
(a) the dyed keratin-containing fibres are re-dyed with a cosmetic agent having an acidic pH and
(b) optionally after a period of up to 4 weeks are again re-dyed with a cosmetic agent having an alkaline pH.

13. Method according to claim 12, **characterised in that** the cosmetic agent with an acidic pH is not rinsed out of the hair.

14. Method according to one of claims 12 or 13, **characterised in that** the cosmetic agent with an alkaline pH is not rinsed out of the hair.

15. Method according to one of claims 12 to 14, **characterised in that** the cosmetic agent with an acidic pH has a pH of pH 2 to pH 6.

16. Method according to one of claims 12 to 15, **characterised in that** the cosmetic agent with an acidic pH does not comprise a coloring component.

17. Method according to one of claims 12 to 16, **characterised in that** the cosmetic agent with an alkaline pH has a pH of pH 8 to pH 11.

18. Method according to one of claims 12 to 17, **characterised in that** the cosmetic agent with an alkaline pH does not comprise a coloring component.

19. Use of a cosmetic agent with an acidic pH for changing the color of keratin-containing fibres, especially human hair, which had been dyed with a coloring agent, comprising in a cosmetic carrier a combination of components
(A) at least one compound of Formula (I) and/or (II) and/or (III) 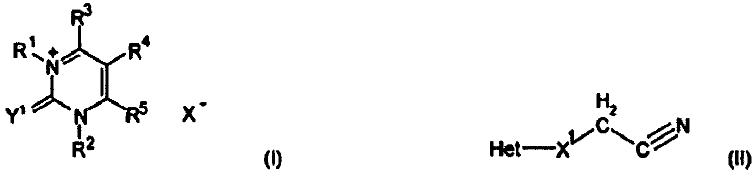 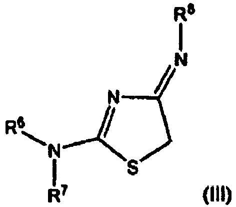 in which R¹, R², R³, R⁴, R⁵, R⁶, R⁷, R⁸, Het, X¹ and Y¹ are as defined in the first Claim
with component (B) at least one compound according to Formula (IV) 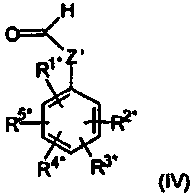 in which R^{1*}, R^{2*} , R^{3*}, R^{4*} , R^{5*} and Z' are as defined in the first Claim.

20. Use of a cosmetic agent with an alkaline pH for restoring a coloration of keratin-containing fibres, especially human hair, which had been initially dyed with a coloring agent, comprising in a cosmetic carrier a combination of components
(A) at least one compound of Formula (I) and/or (II) and/or (III) 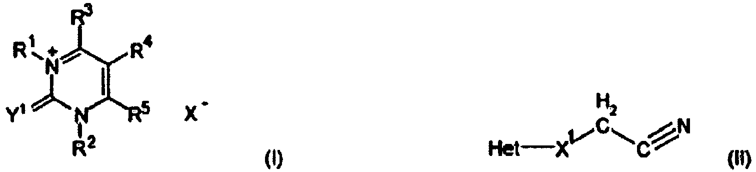 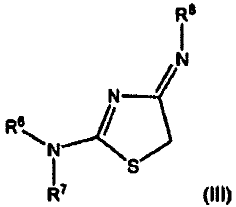 in which R¹, R², R³, R⁴, R⁵, R⁶, R⁷, R⁸, Het, X¹ and Y¹ are as defined in the first Claim
with component (B) at least one compound according to Formula (IV) 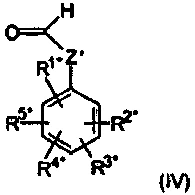 in which R^{1*}, R^{2*} , R^{3*}, R^{4*} , R^{5*} and Z' are as defined in the first Claim,
and then subjected to a color modification with a cosmetic agent having acidic pH.

## Revendications

1. Unité d'emballage (kit), contenant
- dans un contenant 1a un agent, contenant dans un support cosmétique au moins un composé acide CH, sélectionné dans le groupe qui est formé par les composés selon la formule I et/ou leurs formes d'énamine, et par les composés de formule (II), et les composés de formule (III) 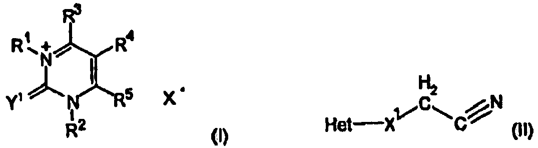 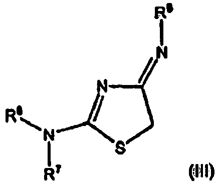 dans lesquelles
• R¹ et R² représentent indépendamment l'un de l'autre un groupe alkyle en C₁ à C₆ linéaire ou cyclique, un groupe alcényle en C₂ à C₆, un groupe aryle éventuellement substitué, un groupe hétéroaryle éventuellement substitué, un groupe aryl(alkyle en C₁ à C₆), un groupe hydroxyalkyle en C₁ à C₆, un groupe polyhydroxyalkyle en C₂ à C₆, un groupe (alcoxy en C₁ à C₆)(alkyle en C₁ à C₆), un groupe R^{I}R^{II}N-(CH₂)ₚ-, dans lequel R^{I} et R^{II} représentent indépendamment l'un de l'autre un atome d'hydrogène, un groupe alkyle en C₁ à C₄, un groupe hydroxyalkyle en C₁ à C₄ ou un groupe aryl(alkyle en C₁ à C₆), dans lequel R^{I} et R^{II} conjointement avec l'atome d'azote peuvent former un cycle à 5, 6 ou 7 membres et p représente un nombre égal à 2, 3, 4, 5 ou 6,
• R³ et R⁵ représentent indépendamment l'un de l'autre un atome d'hydrogène ou un groupe alkyle en C₁ à C₆, dans lesquels au moins l'un des résidus R³ ou R⁵ est un groupe alkyle en C₁ à C₆,
• R⁴ représente un atome d'hydrogène, un groupe alkyle en C₁ à C₆, un groupe hydroxyalkyle en C₁ à C₆, un groupe polyhydroxyalkyle en C₂ à C₆, un groupe alcoxy en C₁ à C₆, un groupe hydroxyalcoxy en C₁ à C₆, un groupe R^{III}R^{IV}N-(CH₂)_{q}-, dans lequel R^{III} et R^{IV} représentent indépendamment l'un de l'autre un atome d'hydrogène, un groupe alkyle en C₁ à C₆, un groupe hydroxyalkyle en C₁ à C₆ ou un groupe aryl(alkyle en C₁ à C₆) et q représente un nombre égal à 1, 2, 3, 4, 5 ou 6, dans lequel le résidu R⁴ conjointement avec l'un des résidus R³ ou R⁵ peut former un cycle aromatique à 5 ou 6 membres, qui peut être éventuellement substitué par un atome d'halogène, un groupe alkyle en C₁ à C₆, un groupe hydroxyalkyle en C₁ à C₆, un groupe polyhydroxyalkyle en C₂ à C₆, un groupe alcoxy en C₁ à C₆, un groupe hydroxyalcoxy en C₁ à C₆, un groupe nitro, un groupe hydroxy, un groupe R^{V}R^{VI}N-(CH₂)ₛ-, dans lequel R^{V} et R^{VI} représentent indépendamment l'un de l'autre un atome d'hydrogène, un groupe alkyle en C₁ à C₆, un groupe hydroxyalkyle en C₁ à C₆ ou un groupe aryl(alkyle en C₁ à C₆) et s représente un nombre égal à 0, 1, 2, 3, 4, 5 ou 6,
• Y¹ représente un atome d'oxygène, un atome de soufre ou un groupe NR^{VII}, dans lequel R^{VII} représente un atome d'hydrogène, un groupe aryle, un groupe hétéroaryle, un groupe alkyle en C₁ à C₆ ou un groupe aryl(alkyle en C₁ à C₆),
• X⁻ représente un anion physiologiquement acceptable,
• Het représente un hétéroaromate éventuellement substitué,
• X1 représente une liaison directe ou un groupe carbonyle,
• R⁶ et R⁷ forment soit conjointement avec l'atome d'azote un cycle à 5 ou 6 membres saturé ou insaturé ou représentent indépendamment l'un de l'autre un groupe alkyle en (C₁ à C₆), un groupe alcényle en (C₂ à C₆), un groupe aryle, un groupe aryl(alkyle en C₁ à C₆), un groupe hydroxyalkyle en (C₂ à C₆), un groupe polyhydroxyalkyle en (C₂ à C₆) ou un groupe R^{I}R^{II}N-(CH₂)ₘ-, dans lequel R^{I} et R^{II} représentent indépendamment l'un de l'autre un atome d'hydrogène, un groupe alkyle (C₁ à C₆), un groupe alcényle en (C₁ à C₆) ou un groupe arylalkyle en C₁ à C₆, dans lequel R^{I} et R^{II} conjointement avec l'atome d'azote peuvent former un cycle à 5, 6 ou 7 membres et m représente un nombre égal à 2, 3, 4, 5 ou 6 et
• R⁸ est un atome d'hydrogène, un groupe alkyle en (C₁ à C₆), un groupe alcényle en (C₂ à C₆), un groupe aryle, un groupe arylalkyle en (C₁ à C₆), un groupe hydroxyalkyle en (C₂ à C₆), un groupe polyhydroxyalkyle en (C₂ à C₆) ou un groupe R^{III}R^{IV}N-(CH₂)ₙ-, dans lequel R^{III} et R^{IV} représentent indépendamment l'un de l'autre un atome d'hydrogène, un groupe alkyle en (C₁ à C₆), un groupe alcényle en (C₂ à C₆) un groupe aryl(alkyle en C₁ à C₆), dans lequel R^{III} et R^{IV} conjointement avec l'atome d'azote peuvent former un cycle à 5, 6 ou 7 membres et n représente un nombre égal à 2, 3, 4, 5 ou 6,
- dans un contenant 1b un agent, contenant dans un support cosmétique au moins un aldéhyde selon la formule (IV) 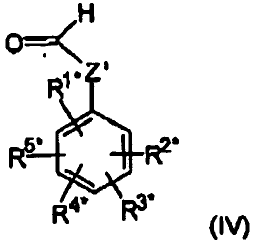 dans laquelle
• R^{1*}, R^{2*} et R^{3*} représentent indépendamment les uns des autres un atome d'hydrogène, un atome d'halogène, un groupe alkyle en C₁ à C₆, un groupe hydroxy, un groupe alcoxy en C₁ à C₆, un groupe dialkylamino en C₁ à C₆, un groupe di(hydroxyalkyle en C₂ à C₆)amino, un groupe di(alcoxy en C₁ à C₆ alkyle en C₁ à C₆)amino, un groupe hydroxyalcoxy en C₁ à C₆, un groupe sulfonyle, un groupe carboxy, un groupe acide sulfonique, un groupe sulfonamido, un groupe sulfonamide, un groupe carbamoyle, un groupe acyle en C₂ à C₆ ou un groupe nitro,
• Z' représente une liaison directe ou un groupe vinylène,
• R^{4*} et R^{5*} représentent un atome d'hydrogène ou forment ensemble, conjointement avec la molécule restante un cycle aromatique ou aliphatique à 5 ou 6 membres,
- dans un contenant 2 un agent cosmétique avec une valeur de pH acide et
- dans un contenant 3 un agent cosmétique avec une valeur de pH alcalin.

2. Kit selon la revendication 1, **caractérisé en ce que** les composés selon la formule 1 sont sélectionnés parmi un ou plusieurs composés du groupe des sels ayant un contre-ion X⁻ physiologiquement acceptable, qui est formé par les sels du
1,2-dihydro-1,3,4,6-tétraméthyl-2-oxopyrimidinium,
1,2-dihydro-1,3-diéthyl-4,6-diméthyl-2-oxopyrimidinium,
1,2-dihydro-1,3-dipropyl-4,6-diméthyl-2-oxopyrimidinium,
1,2-dihydro-1,3-di(2-hydroxyéthyl)-4,6-diméthyl-2-oxopyrimidinium,
1,2-dihydro-1,3-diphényl-4,6-diméthyl-2-oxopyrimidinium,
1,2-dihydro-1,3,4-triméthyl-2-oxopyrimidinium,
1,2-dihydro-1,3-diéthyl-4-méthyl-2-oxopyrimidinium,
1,2-dihydro-1,3-dipropyl-4-méthyl-2-oxopyrimidinium,
1,2-dihydro-1,3-di(2-hydroxyéthyl)-4-méthyl-2-oxopyrimidinium,
1,2-dihydro-1,3-diphényl-4-méthyl-2-oxopyrimidinium,
1-allyl-1,2-dihydro-3,4,6-triméthyl-2-oxopyrimidinium,
1,2-dihydro-1-(2-hydroxyéthyl)-3,4,6-triméthyl-2-oxopyrimidinium,
1,2-dihydro-1,3,4,6-tétraméthyl-2-thioxopyrimidinium,
1,2-dihydro-1,3-diéthyl-4,6-diméthyl-2-thioxopyrimidinium,
1,2-dihydro-1,3-dipropyl-4,6-diméthyl-2-thioxopyrimidinium,
1,2-dihydro-1,3-di(2-hydroxyéthyl)-4,6-diméthyl-2-thioxopyrimidinium,
1,2-dihydro-1,3-diphényl-4,6-diméthyl-2-thioxopyrimidinium,
1,2-dihydro-1,3,4-triméthyl-2-thioxopyrimidinium,
1,2-dihydro-1,3-diéthyl-4-méthyl-2-thioxopyrimidinium,
1,2-dihydro-1,3-dipropyl-4-méthyl-2-thioxopyrimidinium,
1,2-dihydro-1,3-di(2-hydroxyéthyl)-4-méthyl-2-thioxopyrimidinium,
1,2-dihydro-1,3-diphényl-4-méthyl-2-thioxopyrimidinium,
1,2-dihydro-3,4-diméthyl-2-oxo-quinazolinium et
1,2-dihydro-3,4-diméthyl-2-thioxoquinazolinium.

3. Kit selon l'une des revendications 1 ou 2, **caractérisé en ce que** les composés selon la formule (II) sont sélectionnés à partir de composés dans lesquels le résidu Het selon la formule II est dérivé d'un des hétéroatomes furane, thiophène, pyrrole, isoxazole, isothiazole, imidazole, oxazole, thiazole, pyridine, pyridazine, pyrimidine, pyrazine, 1,2,3-triazine, 1,2,4-triazine, 1,3,5-triazine, benzopyrrole, benzofurane, benzothiophène, benzimidazole, benzoxazole, indazole, benzoisoxazole, benzoisothiazole, indole, quinoline, isoquinoline, cinnoline, phtalazine, quinazoline, quinoxaline, acridine, benzoquinoline, benzoisoquinoline, phénazine, benzocinnoline, benzoquinazoline, benzoquinoxaline, phénoxazine, phénothiazine, néphthyridine, phénanthroline, indolizine, quinolizine, carboline, purine, ptéridine et cumarine, dans lesquels lesdits hétéroatomes peuvent être substitués par au moins un groupe sélectionné parmi un atome d'halogène, un groupe nitro, un groupe thio, un groupe thioalkyle en (C₁ à C₆), un groupe hétéroaryle, un groupe aryle, un groupe alkyle en (C₁ à C₆), un groupe alcoxy en (C₁ à C₆), un groupe hydroxy, un groupe hydroxyalkyle en (C₂ à C₆), un groupe polyhydroxyalkyle en (C₂ à C₆), un groupe (alcoxy en C₁ à C₆) (alkyle en C₁ à C₆), un groupe aryl(alkyle en C₁ à C₆), un groupe amino, un groupe monoalkylamino en (C₁ à C₆), un groupe dialkylamino en (C₁ à C₆), un groupe dialkylaminoalkyle -(CH₂)ₙ-NR'R", dans lequel n est un nombre entier allant de 2 à 6 et R' et R" sont indépendamment l'un de l'autre un groupe alkyle linéaire ou ramifié, qui peuvent former éventuellement ensemble un cycle.

4. Kit selon l'une des revendications 1 à 3, **caractérisé en ce que** les composés selon la formule II sont sélectionnés parmi au moins un élément du groupe constitué par le 2-(2-furoyl)-acétonitrile, le 2-(5-bromo-2-furoyl)-acétonitrile, le 2-(5-méthyl-2-trifluorométhyl-3-furoyl)-acétonitrile, le 3-(2,5-diméthyl-3-furyl)-3-oxopropanitrile, le 2-(2-thénoyl)-acétonitrile, le 2-(3-thénoyl)-acétonitrile, le 2-(5-fluoro-2-thénoyl)-acétonitrile, le 2-(5-chloro-2-thénoyl)-acétonitrile, le 2-(5-bromo-2-thénoyl)-acétonitrile, le 2-(5-méthyl-2-thénoyl)-acétonitrile, le 2-(2,5-diméthylpyrrol-3-oyl)-acétonitrile, le 2-(1,2,5-triméthylpyrrol-3-oyl)-acétonitrile, le 1H-benzimidazol-2-ylacétonitrile, le 1H-benzothiazol-2-ylacétonitrile, le 2-(pyrid-2-yl)-acétonitrile, la 2,6-bis(cyanométhyl)-pyridine, le 2-(indol-3-oyl)-acétonitrile, le 2-(2-méthyl-indol-3-oyl)-acétonitrile, la 8-cyanoacétyl-7-méthoxy-4-méthylcumarine, le 2-(2-isopropyl-5,6-benzoquinolin-4-oyl)-acétonitrile, le 2-(2-phényl-5,6-benzoquinolin-4-oyl)-acétonitrile, le 2-(quinoxalin-2-yl)-acétonitrile, le 2-(cumaron-2-yl)-acétonitrile, l'ester tert-butylique de l'acide 6,7-dichloro-5-(cyanoacétyl)-2,3-dihydro-1-benzofuran-2-carboxylique, le 2-(6-hydroxy-4,7-diméthoxy-1-benzofuran-5-oyl)-acétonitrile et le 2-(1-phényl-1,4-dihydrothiochroméno[4,3-c]pyrazol-3-oyl)-acétonitrile.

5. Kit selon l'une des revendications 1 à 4, **caractérisé en ce que** les composés de formule (III) sont sélectionnés parmi les éléments dans lesquels les résidus R⁶ et R⁷ selon la formule (III) forment conjointement avec l'atome d'azote un cycle à 5 ou 6 membres saturé.

6. Kit selon l'une des revendications 1 à 5, **caractérisé en ce que** les composés de formules (I), (II) et (III) sont sélectionnés parmi au moins un élément parmi
| | |
|---|---|
| les sels du 1,2-dihydro-1,2,3,4,6-tétraméthyl-2-oxopyrimidinium | 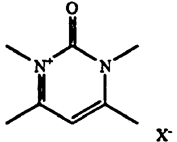 |
| les sels du 1,2-dihydro-1,3,4-triméthyl-2-oxopyrimidinium | 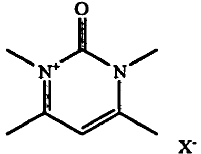 |
| les sels du 1,2-dihydro-1,3,4,6-tétraméthyl-2-thioxopyrimidinium | 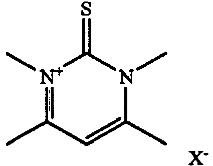 |
| les sels du 1-allyl-1,2-dihydro-3,4,6-triméthyl-2-oxopyrimidinium | 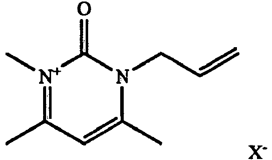 |
| les sels du 1,2-dihydro-1-(2-hydroxyéthyl)-3,4,6-triméthyl-2-oxopyrimidinium | 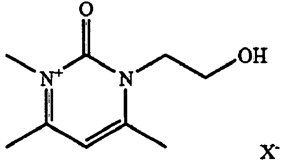 |
| le 2-(cyanométhyl)-benzimidazole | 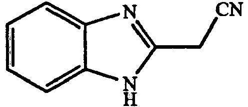 |
| le 4,5-dihydro-4-imino-2-(1-pipéridinyl)-thiazole | 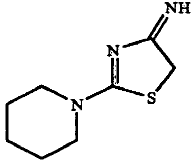 |
| le 4,5-dihydro-4-imino-2-(4-morpholinyl)-thiazole | 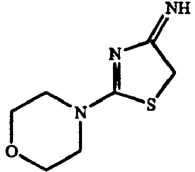 |
| le 4,5-dihydro-4-imino-2-(1-pyrrolidinyl)-thiazole | 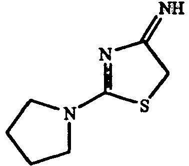 |
dans lequel les sels desdits composés contiennent X⁻ en tant que contre-ion physiologiquement acceptable.

7. Kit selon l'une des revendications 1 à 6, **caractérisé en ce que** les composés de formule (IV) sont sélectionnés parmi au moins un composé du groupe 4-hydroxy-3-méthoxybenzaldéhyde, 3,5-diméthoxy-4-hydroxybenzaldéhyde, 4-hydroxy-1-naphtaldéhyde, 4-hydroxy-2-méthoxybenzaldéhyde, 3,4-dihydroxy-5-méthoxybenzaldéhyde, 3,4,5-trihydroxybenzaldéhyde, 3,5-dibromo-4-hydroxybenzaldéhyde, 4-hydroxy-3-nitrobenzaldéhyde, 3-bromo-4-hydroxybenzaldéhyde, 4-hydroxy-3-méthylbenzaldéhyde, 3,5-diméthyl-4-hydroxybenzaldéhyde, 5-bromo-4-hydroxy-3-méthoxybenzaldéhyde, 4-diéthylamino-2-hydroxybenzaldéhyde, 4-diméthylamino-2-méthoxybenzaldéhyde, 2-méthoxybenzaldéhyde, 3-méthoxybenzaldéhyde, 4-méthoxybenzaldéhyde, 2-éthoxybenzaldéhyde, 3-éthoxybenzaldéhyde, 4-éthoxybenzaldéhyde, 4-hydroxy-2,3-diméthoxybenzaldéhyde, 4-hydroxy-2,5-diméthoxybenzaldéhyde, 4-hydroxy-2,6-diméthoxybenzaldéhyde, 4-hydroxy-2-méthylbenzaldéhyde, 4-hydroxy-2,3-diméthylbenzaldéhyde, 4-hydroxy-2,5-diméthylbenzaldéhyde, 4-hydroxy-2,6-diméthylbenzaldéhyde, 3,5-diéthoxy-4-hydroxybenzaldéhyde, 2,6-diéthoxy-4-hydroxybenzaldéhyde, 3-hydroxy-4-méthoxybenzaldéhyde, 2-hydroxy-4-méthoxybenzaldéhyde, 2-éthoxy-4-hydroxybenzaldéhyde, 3-éthoxy-4-hydroxybenzaldéhyde, 4-éthoxy-2-hydroxybenzaldéhyde, 4-éthoxy-3-hydroxybenzaldéhyde, 2,3-diméthoxybenzaldéhyde, 2,4-diméthoxybenzaldéhyde, 2,5-diméthoxybenzaldéhyde, 2,6-diméthoxybenzaldéhyde, 3,4-diméthoxybenzaldéhyde, 3,5-diméthoxybenzaldéhyde, 2,3,4-triméthoxybenzaldéhyde, 2,3,5-triméthoxybenzaldéhyde, 2,3,6-triméthoxybenzaldéhyde, 2,4,6-triméthoxybenzaldéhyde, 2,4,5-triméthoxybenzaldéhyde, 2,5,6-triméthoxybenzaldéhyde, 2-hydroxybenzaldéhyde, 3-hydroxybenzaldéhyde, 4-hydroxybenzaldéhyde, 2,3-dihydroxybenzaldéhyde, 2,4-dihydroxybenzaldéhyde, 2,4-dihydroxy-3-méthylbenzaldéhyde, 2,4-dihydroxy-5-méthylbenzaldéhyde, 2,4-dihydroxy-6-méthylbenzaldéhyde, 2,4-dihydroxy-3-méthoxybenzaldéhyde, 2,4-dihydroxy-5-méthoxybenzaldéhyde, 2,4-dihydroxy-6-méthoxybenzaldéhyde, 2,5-dihydroxybenzaldéhyde, 2,6-dihydroxybenzaldéhyde, 3,4-dihydroxybenzaldéhyde, 3,4-dihydroxy-2-méthylbenzaldéhyde, 3,4-dihydroxy-5-méthylbenzaldéhyde, 3,4-dihydroxy-6-méthylbenzaldéhyde, 3,4-dihydroxy-2-méthoxybenzaldéhyde, 3,5-dihydroxybenzaldéhyde, 2,3,4-trihydroxybenzaldéhyde, 2,3,5-trihydroxybenzaldéhyde, 2,3,6-trihydroxybenzaldéhyde, 2,4,6-trihydroxybenzaldéhyde, 2,4,5-trihydroxybenzaldéhyde, 2,5,6-trihydroxybenzaldéhyde, 4-diméthylaminobenzaldéhyde, 4-diéthylaminobenzaldéhyde, 4-diméthylamino-2-hydroxybenzaldéhyde, 4-pyrrolidinobenzaldéhyde, 4-morpholinobenzaldéhyde, 2-morpholinobenzaldéhyde, 4-pipéridinobenzaldéhyde, 3,5-dichloro-4-hydroxybenzaldéhyde, 4-hydroxy-3,5-düodobenzaldéhyde, 3-chloro-4-hydroxybenzaldéhyde, 5-chloro-3,4-dihydroxybenzaldéhyde, 5-bromo-3,4-dihydroxybenzaldéhyde, 3-chloro-4-hydroxy-5-méthoxybenzaldéhyde, 4-hydroxy-3-iodo-5-méthoxybenzaldéhyde, 2-méthoxy-1-naphtaldéhyde, 4-méthoxy-1-naphtaldéhyde, 2-hydroxy-1-naphtaldéhyde, 2,4-dihydroxy-1-naphtaldéhyde, 4-hydroxy-3-méthoxy-1-naphtaldéhyde, 2-hydroxy-4-méthoxy-1-naphtaldéhyde, 3-hydroxy-4-méthoxy-1-naphtaldéhyde, 2,4-diméthoxy-1-naphtaldéhyde, 3,4-diméthoxy-1-naphtaldéhyde, 4-diméthylamino-1-naphtaldéhyde, 3-hydroxy-4-nitrobenzaldéhyde, 2-hydroxy-3-méthoxy-5-nitrobenzaldéhyde, 5-nitrovanilline, 2,5-dinitrosalicylaldéhyde, 5-bromo-3-nitrosalicylaldéhyde, 2-diméthylaminobenzaldéhyde, 2-chloro-4-diméthylaminobenzaldéhyde, 4-diméthylamino-2-méthylbenzaldéhyde, 4-diéthylaminocinnamaldéhyde, 4-dibutylaminobenzaldéhyde, 3-allyl-4-hydroxybenzaldéhyde, 3-allyl-4-hydroxy-5-méthoxybenzaldéhyde, 3-allyl-4-hydroxy-5-méthylbenzaldéhyde, 3-allyl-5-bromo-4-hydroxybenzaldéhyde, 3,5-diallyl-4-hydroxybenzaldéhyde, 3-allyl-4-hydroxy-5-formylbenzaldéhyde, (5-allyl-4-hydroxyisophtalaldéhyde) et pipéronal.

8. Kit selon l'une des revendications 1 à 7, **caractérisé en ce que** l'agent cosmétique, ayant une valeur de pH acide possède une valeur de pH allant de pH 2 à pH 6.

9. Kit selon l'une des revendications 1 à 8, **caractérisé en ce que** l'agent cosmétique ayant une valeur de pH acide ne contient aucun composant colorant.

10. Kit selon l'une des revendications 1 à 9, **caractérisé en ce que** l'agent cosmétique ayant une valeur de pH alcalin possède une valeur de pH allant de pH 8 à pH 11.

11. Kit selon l'une des revendications 1 à 10, **caractérisé en ce que** l'agent cosmétique ayant une valeur de pH alcalin ne contient aucun composant colorant.

12. Procédé de recoloration réversible de fibres kératiniques, en particulier des cheveux humains, qui ont auparavant été colorées avec un colorant, contenant dans un support cosmétique une combinaison de composants (A) d'au moins un composé de formule (I) et/ou (II) et/ou (III) 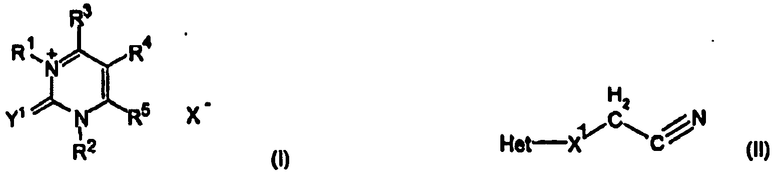 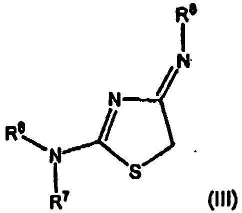 dans lesquelles R¹, R², R³, R⁴, R⁵, R⁶, R⁷, R⁸, Het, X¹ et Y¹ sont tels que définis dans la première revendication
avec un composant (B) d'au moins un composé selon la formule (IV) 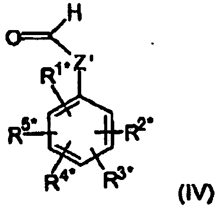 dans laquelle R^{1*}, R^{2*}, R^{3*}, R^{4*}, R^{5*} et Z^{I} sont tels que définis dans la première revendication, et
(a) les fibres colorées kératiniques sont recolorées avec un agent cosmétique ayant une valeur de pH acide et
(b) éventuellement après une période allant jusqu'à 4 semaines elles sont à nouveau recolorées avec un agent cosmétique ayant une valeur de pH alcalin.

13. Procédé selon la revendication 12, **caractérisé en ce que** l'agent cosmétique ayant une valeur de pH acide n'est pas enlevé des cheveux par rinçage.

14. Procédé selon l'une des revendications 12 ou 13, **caractérisé en ce que** l'agent cosmétique ayant une valeur de pH alcalin n'est pas enlevé des cheveux par rinçage.

15. Procédé selon l'une des revendications 12 à 14, **caractérisé en ce que** l'agent cosmétique ayant une valeur de pH acide possède une valeur de pH allant de pH 2 à pH 6.

16. Procédé selon l'une des revendications 12 à 15, **caractérisé en ce que** l'agent cosmétique ayant une valeur de pH acide ne contient aucun composant colorant.

17. Procédé selon l'une des revendications 12 à 16, **caractérisé en ce que** l'agent cosmétique ayant une valeur de pH alcalin possède une valeur de pH allant de pH 8 à pH 11.

18. Procédé selon l'une des revendications 12 à 17, **caractérisé en ce que** l'agent cosmétique ayant une valeur de pH alcalin ne contient aucun composant colorant.

19. Utilisation d'un agent cosmétique ayant une valeur de pH acide pour modifier la couleur de fibres kératiniques, en particulier des cheveux humains, qui ont été colorés avec un colorant, contenant dans un support cosmétique une combinaison de composants
(A) d'au moins un composé de formule (I) et/ou (II) et/ou (III) 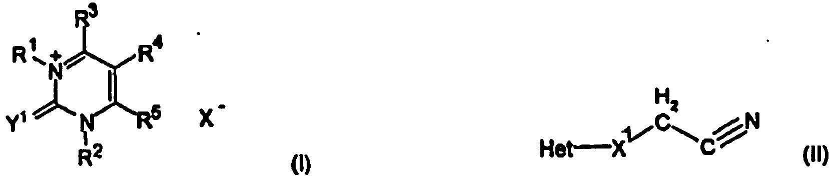 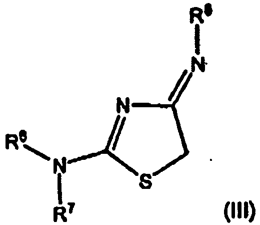 dans lesquelles R¹, R², R³, R⁴, R⁵, R⁶, R⁷, R⁸, Het, X¹ et Y¹ sont tels que définis dans la première revendication
avec un composant (B) d'au moins un composé selon la formule (IV) 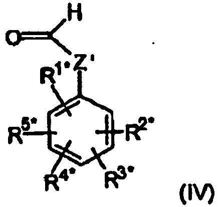 dans laquelle R^{1*}, R^{2*}, R^{3*}, R^{4*}, R^{5*} et Z^{I} sont tels que définis dans la première revendication.

20. Utilisation d'un agent cosmétique ayant une valeur de pH alcalin pour la reconstitution d'une coloration de fibres kératiniques, en particulier des cheveux humains, qui ont d'abord été colorées avec un colorant, contenant dans un support cosmétique une combinaison de composants
(A) d'au moins un composé de formule (I) et/ou (II) et/ou (III) 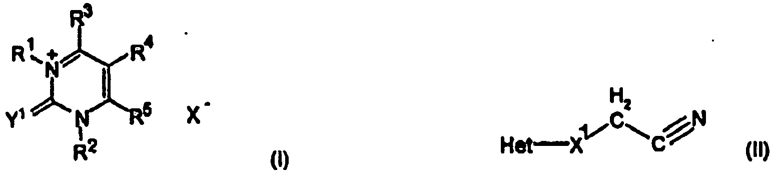 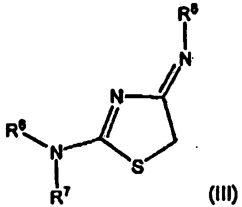 dans lesquelles R¹, R², R³, R⁴, R⁵, R⁶, R⁷, R⁸, Het, X¹ et Y¹ sont tels que définis dans la première revendication
avec un composant (B) d'au moins un composé selon la formule (IV) 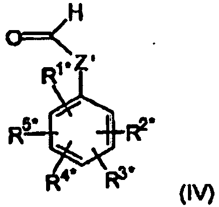 dans laquelle R^{1*}, R^{2*} , R^{3*}, R^{4*} , R^{5*} et Z^{I} sont tels que définis dans la première revendication, et ensuite sont soumises à une modification de la couleur avec un agent cosmétique ayant une valeur de pH acide.
